# EUROPEAN PATENT APPLICATION

(11) **EP 4 563 122 A1**
(43) Date of publication of application: **04.06.2025**
(21) Application number: 24215198.3
(22) Date of filing: 25.11.2024
(51) Int. Cl.: A61F 2/24

(54) **COMMISSURE ALIGNMENT FOR TRANSCATHETER PROSTHETIC HEART VALVES**

(30) Priority: 28.11.2023 US 202363603344 P
(71) Applicant: St. Jude Medical, Cardiology Division, Inc., St. Paul, MN 55117 (US)
(72) Inventor: FINN, Ryan, St. Paul, MN 55113 (US); REIMER, Jay, St. Paul, MN 55101 (US); VIETMEIER, Kristopher Henry, Monticello, MN 55362 (US); NESS, Peter J., Minneapolis, MN 55417 (US); MOORE, Brandon, St. Louis Park, MN 55416 (US)
(74) Representative: LKGLOBAL Lorenz & Kopf Patentanwalt Attorney at Law PartG mbB

(57) **Abstract**

A method of implanting a prosthetic aortic may include advancing a delivery device into the patient until a distal end is positioned within or adjacent to a native aortic valve annulus while the prosthetic aortic is collapsed within the delivery device. While the distal end portion of the delivery device is within or adjacent to the native aortic valve annulus, an alignment wire that is operably coupled to the delivery device may be advanced into a coronary artery and/or an aortic head vessel of the patient. The prosthetic aortic valve may be deployed into the native aortic valve annulus of the patient by expanding the prosthetic aortic valve so that prosthetic commissures of the prosthetic aortic valve rotationally align with native commissures of a native aortic valve of the patient.

## Description

### Cross-Reference to Related Applications

This application claims priority to the filing date of U.S. Provisional Patent Application No, 63/603,344, filed November 28, 2023.

### Background of the Disclosure

The present disclosure generally relates to systems and/or methods for aligning commissures of prosthetic heart valves with native commissures of the heart valve being replaced by the prosthetic heart valve.

Prosthetic heart valves that are collapsible to a relatively small circumferential size can be delivered into a patient less invasively than valves that are not collapsible. For example, a collapsible valve may be delivered into a patient via a tube-like delivery apparatus such as a catheter, a trocar, a laparoscopic instrument, or the like. This collapsibility can avoid the need for a more invasive procedure such as full open-chest, open-heart surgery.

Collapsible prosthetic heart valves typically take the form of a valve structure mounted within a collapsible and expandable frame. There are two types of frames on which the valve structures are typically mounted: a self-expanding frame or a balloon-expandable frame, although other types of expanding frames may be suitable for use in a collapsible prosthetic heart valve. To place such valves into a delivery apparatus and ultimately into a patient, the valve is typically first collapsed or crimped to reduce its circumferential size.

When a collapsed prosthetic valve has reached the desired implant site in the patient (e.g., at or near the annulus of the patient's heart valve that is to be replaced by the prosthetic valve), the prosthetic valve can be deployed or released from the delivery apparatus and expanded (or re-expanded) to full operating size. For balloon-expandable valves, this generally involves expanding a balloon on the delivery device which is positioned inward of the collapsed prosthetic heart valve. For self-expanding valves, on the other hand, the frame automatically expands as the sheath covering the valve is withdrawn.

The present disclosure addresses problems and limitations associated with the related art.

### Summary of the Disclosure

When a prosthetic heart valve is implanted into a native heart valve, it may be desirable for the commissures of the prosthetic heart valve (*e.g.* the areas at which a side of one prosthetic heart valve leaflet meet with a side of an adjacent prosthetic heart valve leaflet) to rotationally align with the native commissures of the native heart valve. If a prosthetic heart valve is being implanted into a previously-implanted prosthetic heart valve (*e.g.* a "valve-in-valve" procedure), it may similarly be desirable for the commissures of the new prosthetic heart valve to align with the commissure of the old prosthetic heart valve, preferably with both sets of prosthetic commissures aligning with the native commissures. Whether the prosthetic heart valve is replacing the function of a native heart valve or a previously-implanted prosthetic heart valve, commissure alignment may help reduce the risk of coronary obstruction, and it may be generally desirable for the prosthetic valve to mimic the native valve anatomy as closely as possible. When implanting prosthetic heart valves surgically (*i.e.* through an open heart, open chest procedure), there is typically good visualization of the operative field, which makes alignment of the prosthetic commissures with the native commissures relatively easy. However, in transcatheter procedures, the entire surgical field is not capable of visualization by the naked eye of the surgeon. For example, deployment of the prosthetic heart valve in a transcatheter procedure is frequently performed under fluoroscopic imaging. Also, the prosthetic heart valve is typically positioned at one end of a delivery device whereas the surgeon is manipulating the opposite end of the delivery device. This can make it significantly more difficult to align the commissures of the prosthetic heart valve with the native valve commissures during a transcatheter heart valve replacement procedure compared to a surgical heart valve replacement procedure. Thus, it would be desirable for systems and methods to help assist with aligning commissures of a prosthetic heart valve with native heart valve commissures, particularly for transcatheter implantation systems.

According to an example of the disclosure, a method of implanting a prosthetic aortic valve into a patient includes advancing a delivery device through a vasculature of the patient until a distal end portion of the delivery device is positioned within or adjacent to a native aortic valve annulus of the patient, the prosthetic aortic valve being mounted to or within the distal end portion of the delivery device in a collapsed condition during the advancing. While the distal end portion of the delivery device is positioned within or adjacent to the native aortic valve annulus of the patient, an alignment wire that is operably coupled to the delivery device may be advanced into a vessel of the patient, the vessel selected from the group consisting of a coronary artery and an aortic head vessel. The prosthetic aortic valve may be deployed into the native aortic valve annulus of the patient by expanding the prosthetic aortic valve so that prosthetic commissures of the prosthetic aortic valve rotationally align with native commissures of a native aortic valve of the patient. The alignment wire may be advanced into the coronary artery, and the coronary artery being selected from the group consisting of a left coronary artery and a right coronary artery. The delivery device may include an aperture in a wall thereof, and advancing the alignment wire may include advancing the alignment wire through the wall of the delivery device and into the coronary artery. The method may also include mounting the prosthetic aortic valve to or within the distal end portion of the delivery device so that, prior to advancing the delivery device through the vasculature, the aperture is positioned circumferentially centered between two of the prosthetic commissures. The alignment wire may be advanced into the aortic head vessel, the aortic head vessel being selected from the group consisting of a brachiocephalic artery, a left common carotid artery, and a left subclavian artery. The delivery device may include an aperture in a wall thereof, and advancing the alignment wire may include advancing the alignment wire through the wall of the delivery device and into the aortic head vessel. The method may include, prior to advancing the delivery device through the vasculature of the patient, (i) determining a rotational offset between an ostium of the aortic head vessel and one of the native commissures, and (ii) mounting the prosthetic aortic valve to or within the distal end portion of the delivery device based on the determined rotational offset so that, when the aperture is rotationally aligned with an ostium of the aortic head vessel, the prosthetic commissures of the prosthetic aortic valve are rotationally aligned with the native commissures of the native aortic valve of the patient. The method may also include rotating the delivery device about a central axis thereof prior to advancing the alignment wire into the vessel of the patient. While the distal end portion of the delivery device is positioned within or adjacent to the native aortic valve annulus of the patient, the distal end portion of the delivery device may be positioned nearer to a right coronary artery of the patient than a left coronary artery of a patient. The vessel of the patient may be the left coronary artery.

According to another example of the disclosure, a prosthetic heart valve system may include a delivery device including a catheter, the catheter including an aperture in a sidewall thereof. The system may include a collapsible and expandable prosthetic heart valve including a frame and a plurality of prosthetic leaflets mounted within the frame, the prosthetic heart valve configured to be mounted on or within the catheter in a collapsed condition for transcatheter delivery to a patient. The system may include an alignment wire configured to be positioned within the catheter during transcatheter deliver to the patient, the alignment wire being formed of a shape memory material and being shape-set so that, while the alignment wire is within the catheter it is substantially straight and after being advanced through the aperture, a distal portion of the alignment wire extending beyond the aperture is biased at an angle of about 90 degrees relative to an intermediate portion of the alignment wire remaining within the catheter. The delivery device may include a handle at a proximal end thereof, and an actuator operatively coupled to the handle, wherein actuation of the actuator rotates the catheter so that, when the prosthetic heart valve is mounted on or within the catheter, rotation of the catheter causes rotation of the prosthetic heart valve. The alignment wire may be formed of nitinol. The alignment wire may be inflatable. The alignment may be ribbon-shaped with a substantially rectangular cross-section. The prosthetic heart valve may be self-expanding, and the catheter may include an outer shaft and a distal sheath positioned distal to the outer shaft, the distal sheath having a diameter that is larger than a diameter of the outer shaft, the prosthetic heart valve configured to be mounted within the distal sheath of the catheter during transcatheter delivery to the patient. The aperture may be positioned on the distal sheath so that when a portion of the distal sheath is positioned within a native aortic valve annulus of the patient, the aperture is substantially axially aligned with an ostium of a left coronary artery or an ostium of a right coronary artery. The aperture may be positioned on the outer shaft so that when a portion of the distal sheath is positioned within a native aortic valve annulus of the patient, the aperture is substantially axially aligned with an ostium of a brachiocephalic artery, an ostium of a left common carotid artery, or an ostium of a left subclavian artery. The sidewall of the catheter may include a lumen extending axially therethrough, the alignment wire being configured to be received through the lumen. The lumen may terminate in the aperture.

According to a further example of the disclosure, a prosthetic heart valve system may include a delivery device including a catheter, the catheter including a distal end portion and a plurality of apertures in a sidewall thereof. The system may include a collapsible and expandable prosthetic heart valve including a frame and a plurality of prosthetic leaflets mounted within the frame, the prosthetic heart valve configured to be mounted on or within the distal end portion of the catheter in a collapsed condition for transcatheter delivery to a patient. The system may include a plurality of alignment wires configured to be positioned within the catheter during transcatheter deliver to the patient, the plurality of alignment wires each configured to be substantially straight when received within the catheter and to form a distal looped end after extending beyond a respective one of the plurality of apertures. The distal end portion of the catheter may be rotatable about a central longitudinal axis thereof, such that upon rotation of the distal end portion of the catheter about the central longitudinal axis when the prosthetic heart valve is mounted on or within the distal end portion of the catheter, the prosthetic heart valve and the plurality of alignment wires also rotate about the central longitudinal axis. The prosthetic heart valve may be a prosthetic aortic valve, and the plurality of alignment wires may include three alignment wires, and the plurality of apertures may include three apertures. The distal looped end of each of the three alignment wires may be sized and shaped to be received within a nadir of a corresponding one of three native aortic valve leaflets. The distal looped end of each of the three alignment wires may include two wire portions that each loop or hook proximally and meet at an apex to form a recess, each recess being sized and shaped to be received over a corresponding one of three native aortic valve commissures. The distal end portion of the catheter may be passively rotatable about the central longitudinal axis, such that after the three alignment wires exit the three apertures to form the distal looped ends, contact between the distal looped ends and a native aortic valve of the patient causes the distal end portion of the catheter to passively rotate about the central longitudinal axis.

According to still another example of the disclosure, a method of implanting a prosthetic aortic valve into a patient may include advancing a delivery device through a vasculature of the patient until a distal end portion of the delivery device is positioned within or adjacent to a native aortic valve annulus of the patient, the prosthetic aortic valve being mounted to or within the distal end portion of the delivery device in a collapsed condition during the advancing. While the distal end portion of the delivery device is positioned within or adjacent to the native aortic valve annulus of the patient, an alignment wire operably coupled to the delivery device may be advanced to a position outside of the delivery device. The distal end portion of the delivery device may be rotated about a central longitudinal axis thereof and contacting the alignment wire with tissue of a native aortic valve of the patient. After the rotating and contacting, the prosthetic aortic valve may be deployed into the native aortic valve annulus of the patient by expanding the prosthetic aortic valve so that prosthetic commissures of the prosthetic aortic valve rotationally align with native commissures of a native aortic valve of the patient. After the alignment wire advances to the position outside of the delivery device, it may form a looped distal end, and contacting the alignment wire with tissue may include positioning the looped distal end of the alignment wire within a nadir of a leaflet of the native aortic valve of the patient. After contacting the alignment wire with tissue of the native aortic valve, the distal looped end of the alignment wire may be advanced toward the nadir of the leaflet as the distal end portion of the delivery device passively rotates about the central longitudinal axis. After the alignment wire advances to the position outside of the delivery device, it may form a looped distal end, the looped end including two wire portions that each loop or hook proximally and meet at an apex to form a recess. Contacting the alignment wire with tissue of the native aortic valve of the patient may include positioning one of the native commissures of the native aortic valve within the recess.

According to a further example of the disclosure, a method of implanting a prosthetic aortic valve into a patient may include advancing a delivery device through a vasculature of the patient until a distal end portion of the delivery device is positioned within or adjacent to a native aortic valve annulus of the patient, the prosthetic aortic valve being mounted to or within the distal end portion of the delivery device in a collapsed condition during the advancing. While the distal end portion of the delivery device is positioned within or adjacent to the native aortic valve annulus of the patient, an alignment wire that is operably coupled to the delivery device may be advanced into contact with tissue of a native mitral valve of the patient. The prosthetic aortic valve may be deployed into the native aortic valve annulus of the patient by expanding the prosthetic aortic valve so that prosthetic commissures of the prosthetic aortic valve rotationally align with native commissures of a native aortic valve of the patient. The distal end portion of the delivery device may be rotated about a central longitudinal axis thereof, and advancing the alignment wire into contact with tissue of the native mitral valve may include contacting a distal end of the alignment wire with a base of an anterior leaflet of the native mitral valve on an outflow side of the anterior leaflet. Prior to advancing the delivery device through the vasculature of the patient, (i) a rotational offset between the base of the anterior leaflet and one of the native commissures may be determined, and (ii) the prosthetic aortic valve may be mounted to or within the distal end portion of the delivery device based on the determined rotational offset so that, when the distal end of the alignment wire contacts the base of the anterior leaflet, the prosthetic commissures of the prosthetic aortic valve are rotationally aligned with the native commissures of the native aortic valve of the patient. The alignment wire may include a first alignment wire and a second alignment wire. The distal end portion of the delivery device may be rotated about a central longitudinal axis thereof, and the alignment wire may be advanced into contact with tissue of the native mitral valve includes contacting a distal end of the first alignment wire with a first native mitral valve commissure of the patient, and a distal end of the second alignment wire may be contacted with a second native mitral valve commissure of the patient. Prior to advancing the delivery device through the vasculature of the patient, (i) a rotational offset between the first native mitral valve commissure of the patient and one of the native commissures of the native aortic valve of the patient may be determined, and (ii) the prosthetic aortic valve may be mounted to or within the distal end portion of the delivery device based on the determined rotational offset so that, when the distal end of the first alignment wire contacts the first native mitral valve commissure and the distal end of the second alignment wire contacts the second native mitral valve commissure, the prosthetic commissures of the prosthetic aortic valve are rotationally aligned with the native commissures of the native aortic valve of the patient. Advancing the alignment wire into contact with tissue of the native mitral valve may include advancing the alignment wire through the native mitral valve and into a left atrium of a patient until the alignment wire contacts an inflow surface of an anterior leaflet of the native mitral valve. Prior to advancing the alignment wire, the distal end portion of the delivery device may be positioned closer to a right coronary cusp of the native aortic valve than a left coronary cusp of the native aortic valve. After the alignment wire contacts the inflow surface of the anterior leaflet of the native mitral valve, the distal end portion of the delivery device may be pulled toward a radial center of the native aortic valve. The distal end portion of the delivery device may be rotated about a central longitudinal axis thereof.

According to another example of the disclosure, a prosthetic heart valve system may include a delivery device including a catheter, and a collapsible and self-expandable prosthetic heart valve configured to be received within a distal end of the catheter so that the distal end of the catheter maintains the prosthetic heart valve in a collapsed condition. The prosthetic heart valve may include a collapsible and self-expandable frame extending between an inflow end and an outflow end, and a prosthetic valve assembly mounted within the frame, the frame including an inflow-most row of cells. The frame may further include a plurality of commissure alignment features, and each of the commissure alignment features may be directly coupled to selected cells in the inflow-most row of cells. In the absence of applied forces, the commissure alignment features may extend farther away from a central longitudinal axis of the frame than do the selected cells. The plurality of commissure alignment features may include three commissure alignment features space substantially evenly around a circumference of the prosthetic heart valve, and each commissure alignment feature may have a pre-determined rotational orientation relative to a corresponding commissure of the prosthetic valve assembly. The pre-determined rotational orientation may be such that each of the three commissure alignment features is longitudinally aligned with the corresponding commissure of the prosthetic valve assembly. Each of the three commissure alignment features may include two struts that each have a first end coupled to a corresponding one of the selected cells, and a second end, the second ends of the two struts joining to form an apex. The first end of each of the two struts may be coupled to a strut of the corresponding one of the selected cells at a midpoint of the strut. Each of the three commissure alignment features may terminate at the apex. Each of the three commissure alignment features may include a circumferentially extending strut coupled to the apex. Each of the three commissure alignment features may include a coil coupled to the apex. The coil may be formed as a wire that extends from the apex and which is coiled into one or more loops. The coil may loop around a coil axis that extends in a direction that is transverse to the central longitudinal axis of the frame.

According to still another example of the disclosure, a method of implanting a self-expanding prosthetic heart valve into a patient may include advancing a delivery device through a vasculature of the patient until a distal end portion of the delivery device is positioned within or adjacent to a native heart valve of the patient, the prosthetic heart valve being mounted to or within the distal end portion of the delivery device in a collapsed condition during the advancing. The prosthetic heart valve may include a collapsible and self-expandable frame including an inflow-most row of cells, and the frame may further include a plurality of commissure alignment features directly coupled to selected cells in the inflow-most row of cells. The method may include starting to retract the distal end portion of the delivery device relative to the prosthetic heart valve to allow an inflow end of the prosthetic heart valve, including the plurality of commissure alignment features, to begin to self-expand while an outflow end of the prosthetic heart valve remains collapsed within the distal end portion of the delivery device. After allowing the inflow end of the prosthetic heart valve begin to self-expand, a position of the plurality of commissure alignment features may be determined with respect to native commissures of the native heart valve under imaging. The distal end portion of the delivery device may be rotated to rotate the prosthetic heart valve and the plurality of commissure alignment features based on the determined position. After rotating the distal end portion of the delivery device, Retraction of the distal end portion of the delivery device relative to the prosthetic heart valve may be continued to allow the prosthetic heart valve to fully expand into the native heart valve such that prosthetic commissures of the prosthetic heart valve rotationally align with the native commissures of the native heart valve of the patient. The plurality of commissure alignment features may be longitudinally aligned with corresponding ones of the prosthetic commissures. Rotating the distal end portion of the delivery device may include rotating the prosthetic heart valve until the plurality of commissure alignment features are rotationally aligned with the corresponding ones of the native commissures. Allowing the prosthetic heart valve to fully expand into the native heart valve may include allowing the plurality of commissure alignment features to contact corresponding ones of the native commissures. Upon allowing the inflow end of the prosthetic heart valve to begin to self-expand, the plurality of commissure alignment features may be positioned farther away from a central longitudinal axis of the frame than any other portion of the frame. Each of the plurality of commissure alignment features may include two struts that each have a first end coupled to a corresponding one of the selected cells, and a second end, the second ends of the two struts joining to form an apex. Each of the plurality of commissure alignment features may terminate at the apex. Each of the plurality of commissure alignment features may include a circumferentially extending strut coupled to the apex. Each of the plurality of commissure alignment features may include a coil coupled to the apex. The coil may be formed as a wire that extends from the apex and which is coiled into one or more loops.

### Brief Description of the Drawings

Fig. 1 is a side elevational view of an example of a collapsible prosthetic heart valve in an expanded condition, showing the valve assembly attached to the stent.
Fig. 2A is side view of an example of an operating handle for a transfemoral delivery device for a collapsible prosthetic heart valve, shown with a side elevational view of the distal portion of a transfemoral catheter assembly.
Fig. 2B is an enlarged view of a distal end of an example in which the prosthetic heart valve of Fig. 1 is maintained in a collapsed condition within the distal sheath of the delivery device of Fig. 2A.
Fig. 3A is a highly schematic cutaway view of an example of a distal end of a delivery device, which may be the delivery device of Fig. 2A or an alternate example thereof, within the aortic arch with a wire cannulating the brachiocephalic artery.
Fig. 3B is a highly schematic cutaway view of an example of a distal end of a delivery device, which may be the delivery device of Fig. 2A or an alternate example thereof, within the aortic valve with a wire cannulating the right coronary artery.
Fig. 3C is a top view of the configuration shown in Fig. 3B.
Fig. 3D is a flow chart showing examples of methods of use relating to Figs. 3A-3C.
Fig. 4A is side view of an example of a distal end of a delivery device, which may be the delivery device of Fig. 2A or an alternate example thereof, with three alignment wires in a deployed confirmation.
Fig. 4B is a top view of the three deployed alignment wires of Fig. 4A engaging a native heart valve.
Fig. 4C is a side view of a distal end of a delivery device with three alignment wires having an alternative configuration to those of Fig. 4A in a deployed condition.
Fig. 4D is schematic side view of an example of the distal end of the delivery device of Fig. 2A with an example of an added joint.
Fig. 4E is a flow chart showing examples of methods of use relating to Figs. 4A-4D.
Fig. 5A is a cutaway view of an example of a delivery device, which may be the delivery device of Fig. 2A or an alternate example thereof, with two alignment fins positioned within an open aortic valve.
Fig. 5B is a cutaway view of an example of a delivery device, which may be the delivery device of Fig. 2A or an alternate example thereof, with three alignment fins positioned within an open aortic valve.
Fig. 5C is a flow chart showing examples of methods of use relating to Figs. 5A-5B.
Fig. 6A is a highly schematic cutaway side view of the left heart with an example of a delivery device, which may be the delivery device of Fig. 2A or an alternate example thereof, positioned within an aortic valve.
Figs. 6B-6C illustrate a highly schematic top view of the aortic valve AV and its position next to the mitral valve MV with other structures of the heart omitted for clarity.
Fig. 6D is a highly schematic cutaway side view of the left heart with an example of a delivery device, which may be the delivery device of Fig. 2A or an alternate example thereof, positioned within an aortic valve.
Fig. 6E is a flow chart showing an example of a method of use relating to Figs. 6A-6B.
Fig. 6F is a flow chart showing an example of a method of use relating to Fig. 6C.
Fig. 6G is a flow chart showing an example of a method of use relating to Fig. 6D.
Fig. 7A is a side view of an example of a prosthetic heart valve in an expanded condition.
Fig. 7B is an enlarged view of an inflow end portion of the frame of the prosthetic heart valve of Fig. 7A.
Fig. 7C is an end view of the inflow end of the frame of the prosthetic heart valve of Fig. 7A.
Figs. 7D-7E are different views of the inflow end of the prosthetic heart valve of Figs. 7A-7C in an early stage of deployment from a delivery device, which may be the delivery device of Fig. 2A or an alternate example thereof,.
Fig. 7F is a view of the inflow end of the prosthetic heart valve of Fig. 7D-7E in a further stage of deployment from the delivery device.
Fig. 7G is a highly schematic representation of the deployment view of Fig. 7F within a native aortic valve.
Figs. 8A-8B are views of an alternate version of the commissure alignment features of Figs. 7A-7C.
Figs. 9A-9B are views of another alternate version of the commissure alignment features of Figs. 7A-7C.
Fig. 10 is a flow chart showing an example of a method of use relating to Figs. 7A-9B.

### Detailed Description of the Disclosure

As used herein in connection with prosthetic heart valves, the term "inflow end" refers to the end of the heart valve through which blood first flows when implanted in an intended position and orientation, while the term "outflow end" refers to the opposite end, through which blood last flows when the prosthetic heart valve is implanted in the intended position and orientation. For example, when used in the context of a prosthetic aortic valve, the "inflow" end refers to the end closer to the left ventricle while the "outflow" end refers to the end closer to the aorta. When used in connection with devices for delivering a prosthetic heart valve into a patient, the terms "proximal" and "distal" are to be taken as relative to the user of the delivery devices. In other words, in this context, "proximal" is to be understood as relatively close to the user of the delivery device (e.g., the "trailing" end), and "distal" is to be understood as relatively farther away from the user of the delivery device (e.g., the "leading" end).

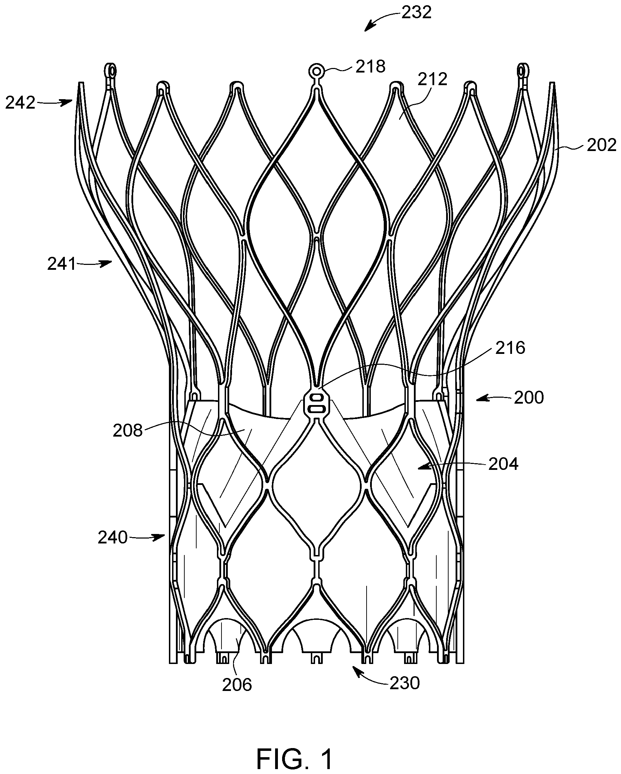

Fig. 1 shows one example of a collapsible prosthetic heart valve 200. The particular example of prosthetic heart valve 200 shown in Fig. 1 is designed to replace the function of a native aortic valve of a patient, although it should be understood that the concepts described herein may be applicable to the replacement of any native heart valve, including the mitral, tricuspid, or pulmonary heart valves. As discussed in detail below, prosthetic heart valve 200 has an expanded condition, shown in Fig. 1, and a collapsed condition.

Prosthetic heart valve 200 may in one example include a collapsible and expandable frame 202 (which may also be referred to as a "stent") that may be formed from any biocompatible material, such as metals, metal alloys, synthetic polymers or biopolymers capable of functioning as a frame. Frame 202 may extend from an inflow or annulus end 230 to an outflow or aortic end 232, and may include an annulus section 240 adjacent the inflow end and an aortic section 242 adjacent the outflow end. In some examples, the annulus section 240 has a relatively small cross-section in the expanded condition, while in some examples, the aortic section 242 has a relatively large cross-section in the expanded condition. Preferably, annulus section 240 is in the form of a cylinder having a substantially constant diameter along its length. However, in other examples, annulus section 240 may have other shapes when expanded, including frustoconical, bulbous, *etc.* A transition section 241 in some examples tapers outwardly from the annulus section 240 to the aortic section 242. Each of the sections of the frame 202 may include a plurality of cells 212 connected to one another in one or more annular rows around the frame, although other configurations may be suitable in other examples. As shown in the example of Fig. 1, the annulus section 240 may have two annular rows of complete cells 212 and the aortic section 242 and transition section 241 may each have one or more annular rows of cells 212. The cells 212 in the aortic section 242 may be larger than the cells 212 in the annulus section 240 in some examples. The larger cells in the aortic section 242 may better enable the prosthetic valve 200 to be positioned without the frame structure interfering with blood flow to the coronary arteries. In the illustrated example, cells 212 are generally diamond-shaped when the frame 202 is in the expanded condition, but it should be understood that the cells in other examples may have other shapes (*e.g.,* chevrons, hexagons, *etc*.)*.*

In some examples, frame 202 includes one or more retaining elements 218 at the outflow end 232 thereof, the retaining elements being sized and shaped to cooperate with complementary retaining structures provided on the deployment device. When included, the engagement of retaining elements 218 with the complementary retaining structures on the deployment device may help maintain prosthetic heart valve 200 in an assembled relationship with the deployment device, may minimize longitudinal (*e.g.* axial) movement of the prosthetic heart valve relative to the deployment device during unsheathing or re-sheathing procedures, and/or may help prevent rotation of the prosthetic heart valve relative to the deployment device as the deployment device is advanced to the target location and during deployment.

The example of prosthetic heart valve 200 illustrated in Fig. 1 includes a valve assembly 204 positioned partially or entirely within the annulus section 240. Valve assembly 204 in some examples may include a skirt or cuff 206 and a plurality of prosthetic leaflets 208 which collectively function as a one-way valve. The commissures between adjacent prosthetic leaflets 208 may be connected to corresponding commissure features 216 on frame 202. The example of prosthetic heart valve 200 shown in Fig. 1 includes three prosthetic leaflets 208, as well as three commissure features 216. As can be seen in Fig. 1, the commissure features 216 may lie at the intersection of four cells 212, two of the cells being adjacent one another in the same annular row, and the other two cells being in different annular rows and lying in end-to-end relationship. However, in other examples, commissure features 216 may be provided at different locations or in different configurations, or even omitted entirely. In some examples, commissure features 216 are positioned entirely within annulus section 240 or at the juncture of annulus section 240 and transition section 241. In the illustrated example, commissure features 216 include one or more eyelets which facilitate the suturing of the leaflet commissure to the frame 202. However, it should be appreciated that the prosthetic heart valve may have more or fewer prosthetic leaflets and commissure features. For example, an alternative prosthetic heart valve (*e.g.,* a prosthetic mitral valve) may include two prosthetic leaflets with two commissures. Additionally, although cuff 206 is shown in Fig. 1 as being disposed on the luminal surface of annulus section 240, it should be understood that the cuff may be disposed on the abluminal surface of annulus section 240, or may cover all or part of either or both of the luminal and abluminal surfaces of annulus section 240. Both the cuff 206 and the leaflets 208 may be wholly or partly formed of any suitable biological material or polymer.

It should be understood that prosthetic heart valve 200 is merely one example of a prosthetic heart valve, and is illustrated as an example to provide better context for the commissure alignment features described in greater detail below. It should be understood that the commissure alignment features described below may be suitable for use in prosthetic heart valves similar to prosthetic heart valve 200, as well as other prosthetic heart valves that are not similar to prosthetic heart valve 200. For example, prosthetic heart valve 200 may be a self-expanding prosthetic heart valve, which may include a frame 202 formed of a shape-memory alloy such as a nickel-titanium alloy (including nitinol). However, unless explicitly described otherwise, the commissure alignment features described below may be suitable for plastically expandable prosthetic heart valves, including those with frames formed of materials such as stainless steel or cobalt chromium, as well as mechanically expandable prosthetic heart valves (*e.g.* those which may be expanded via active foreshortening of the valve frame).

In operation, a prosthetic heart valve, including prosthetic heart valve 200 described above, may be used to replace a native heart valve, such as the aortic valve, a surgical heart valve or a heart valve that has undergone a surgical procedure. The prosthetic heart valve may be delivered to the desired site (*e.g.,* near a native aortic valve annulus) using any suitable delivery device, including the delivery devices described in detail below. During delivery, the prosthetic heart valve is disposed on or inside the delivery device in the collapsed condition. The delivery device may be introduced into a patient using a transfemoral, transapical or transseptal approach, although any transcatheter approach may be suitable. Once the delivery device has reached the target site, the user may deploy the prosthetic heart valve. Upon deployment, the prosthetic heart valve expands into secure engagement within the native aortic valve annulus. When the prosthetic heart valve is properly positioned inside the heart, it works as a one-way valve, allowing blood to flow in one direction and preventing blood from flowing in the opposite direction.

In some examples of a prosthetic aortic heart valve, the valve assembly may be spaced from the outflow or aortic end of the stent by a distance that enables deployment of the heart valve by an amount sufficient for the valve leaflets of the prosthetic valve to operate as intended, while the outflow end of the stent remains captured by the delivery device. More particularly, the inflow or annulus end of the prosthetic heart valve may be deployed first, while the aortic or outflow end of the prosthetic heart valve remains at least partially covered by a distal sheath of the delivery device. The annulus portion of the prosthetic heart valve may be deployed so that the entirety of the valve leaflets, up to and including the commissures, is deployed and fully operational. By deploying the prosthetic heart valve in this manner, the user can determine whether the valve leaflets are properly positioned (*e.g.,* at the desired depth) relative to the native valve annulus, and whether the valve is functioning properly. If the user determines that the positioning and operation of the valve are acceptable, the remainder of the valve may be deployed. However, if it is determined that the leaflet position is improper or that the valve is not functioning properly, the user may re-sheathe the valve and either reposition it for redeployment, or remove it entirely from the patient. This partial deployment and valve testing may be particularly applicable to self-expanding prosthetic heart valves, such as prosthetic heart valve 200. However, it should be understood that the commissure alignment features described below may be implemented whether or not the prosthetic heart valve system has the re-sheathing capabilities described above.

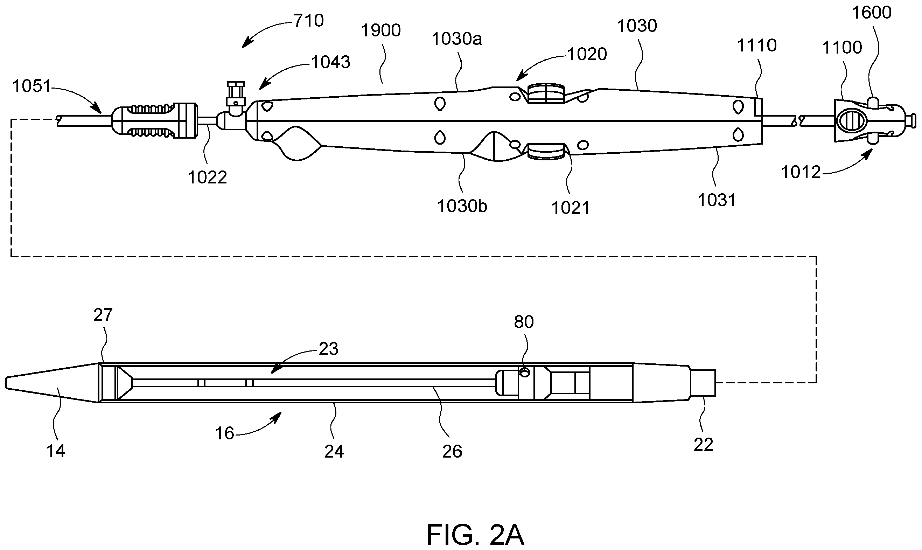

Turning now to Fig. 2A, an exemplary transfemoral delivery device 1010 for a collapsible prosthetic heart valve (or other types of collapsible stents) may have a catheter assembly 1016 for delivering the prosthetic heart valve to and deploying the prosthetic heart valve at a target location, and an operating handle 1020 for controlling deployment of the valve from the catheter assembly. The delivery device 1010 may extend from a proximal end 1012 to a distal tip 1014. In the illustrated example, the catheter assembly 1016 is adapted to receive a collapsible prosthetic heart valve (not shown) in a compartment 1023 defined around an inner shaft 1026 and covered by a distal sheath 1024 (which may also be referred to as a "delivery capsule"). In some examples, the inner shaft 1026 extends through the operating handle 1020 to the distal tip 1014 of the delivery device, and includes a proximal hub and/or retainer 1025 affixed thereto at a spaced distance from distal tip 1014 and adapted to hold a collapsible prosthetic valve in the compartment 1023. The inner shaft 1026 may define, at least in part, a guidewire lumen configured to receive a guidewire therethrough.

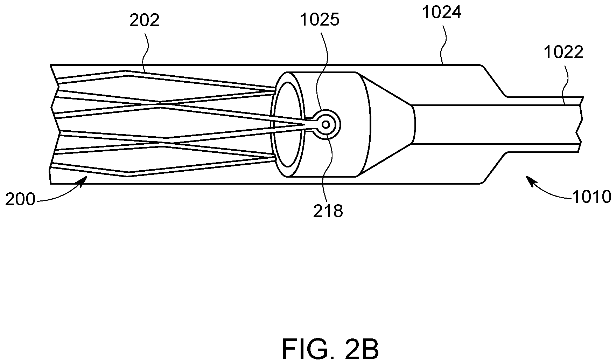

Referring in addition to Fig. 2B, Fig. 2B illustrates an example of the prosthetic heart valve 200 of Fig. 1 being maintained in a collapsed condition within the distal sheath 1024 of the delivery device 1010 of Fig. 2A. In the example of Fig. 2B, for purpose of clarity of illustration, only the atrial or inflow portion of prosthetic heart valve 200 is shown, and the inner shaft 1026 of delivery device 1010 is omitted. The example of Fig. 2B shows one configuration in which retaining elements 218 of the prosthetic heart valve 200 are secured within the retainer 1025 of the delivery device 1010, although it should be understood that other retainer configurations may be suitable.

Referring again to Fig. 2A, in the illustrated example, the distal sheath 1024 surrounds the inner shaft 1026 and is slidable relative to the inner shaft such that it can selectively cover or uncover the compartment 1023. In some examples, the distal sheath 1024 may be affixed at its proximal end to an outer shaft 1022, the proximal end of which may be connected to the operating handle 1020. The distal end 1027 of the distal sheath 1024 may abut the distal tip 1014 when the distal sheath fully covers the compartment 1023, and may be spaced apart from the distal tip 1014 when the compartment 1023 is at least partially uncovered.

The operating handle 1020 in the illustrated example is adapted to control deployment of a prosthetic valve located in the compartment 1023 by permitting a user to selectively slide the outer shaft 1022 proximally or distally relative to the inner shaft 1026, or to slide the inner shaft 1026 relative to the outer shaft 1022, thereby respectively uncovering or covering the compartment with the distal sheath 1024. In some examples, operating handle 1020 may include frame 1030 which extends from a proximal end 1031 to a distal end and may include a top frame portion 1030a and a bottom frame portion 1030b. The proximal end of the inner shaft 1026 may in some examples be coupled to a hub 1100, and the proximal end of the outer shaft 1022 may in some examples be affixed to a carriage assembly within the frame 1030 that is slidable within the operating handle along a longitudinal axis of the frame 1030, such that a user can selectively slide the outer shaft relative to the inner shaft by sliding the carriage assembly relative to the frame. In examples that include the hub 1100, inner shaft 1026 may be actuated via hub 1100 to cover or uncover the compartment, for example for rapid covering or uncovering of the compartment 1023. Optionally, a stability sheath and/or integrated introducer 1051 is disposed over some or all of outer shaft 1022. The stability sheath and/or integrated introducer 1051 may be attached to the outer shaft 1022 or may be unattached. Additionally, stability sheath and/or integrated introducer 1051 may be disposed over a majority of outer shaft 1022 or over a minority of the outer shaft (e.g., over 49% or less, over 33%, etc.). Optionally, stability sheath and/or integrated introducer 1051 may be more rigid than outer shaft 1022.

Additionally, in examples that include the hub 1100, the hub 1100 may include a pair of buttons, each attached to a clip. These clips on hub 1100 may mate with voids or recessed areas on frame 1030 to ensure that the hub and the frame are securely coupled together. Optionally, hub 1100 may also include a wheel 1600 which may assist in reducing strain in the distal sheath 1024 when loading the prosthetic heart valve into the delivery device 1010.

The delivery device in some examples includes a re-sheathing lock 1043, which may restrict motion of the distal sheath 1024 once full deployment of the prosthetic heart valve is imminent. The re-sheathing lock 1043 may be disengaged when the desired position of the prosthetic heart valve is confirmed, so that the distal sheath 1024 may be further retracted to full release the prosthetic heart valve. In other words, the re-sheathing lock 1043 may help prevent unintentional or premature complete deployment of the prosthetic heart valve. Additional features of the delivery device 1010, for example including the function of wheel 1600, are described in greater detail in U.S. Patent Publication No. 2018/0153693.

Although one particular example of a delivery device 1010 is illustrated and described, it should be understood that similar to prosthetic heart valve 200, delivery device 1010 is merely one example of a delivery device that may be suitable for use with a prosthetic heart valve, and the description is intended to provide better context for the commissure alignment features described below. Other delivery devices, including ones with expandable balloons for balloon-expandable prosthetic heart valves, may be just as suitable for use with the commissure alignment features described herein.

As noted above, it may be desirable for the commissures of the prosthetic heart valve to align rotationally with the commissures of the native heart valve upon deployment and /or implantation of the prosthetic heart valve into the native heart valve. As used herein, the term commissure alignment generally refers to at least one prosthetic commissure (including two or three prosthetic commissures) being substantially rotationally aligned with a corresponding native commissure, including within about 10 degrees or less or within about 5 degrees or less of rotational offset in either rotational direction. It may also be desirable for the prosthetic heart valve to be positioned substantially centered within the native heart valve just prior to deployment Various systems and methods are described below to assist with achieving commissure alignment and/or centering. At least some of these systems and methods may rely on the ability to interact with the patient's anatomy to help maximize the likelihood that, upon deployment of the prosthetic heart valve, the prosthetic commissure are rotationally aligned with the native commissures and/or the prosthetic heart valve is centered within the native heart valve just prior to deployment.

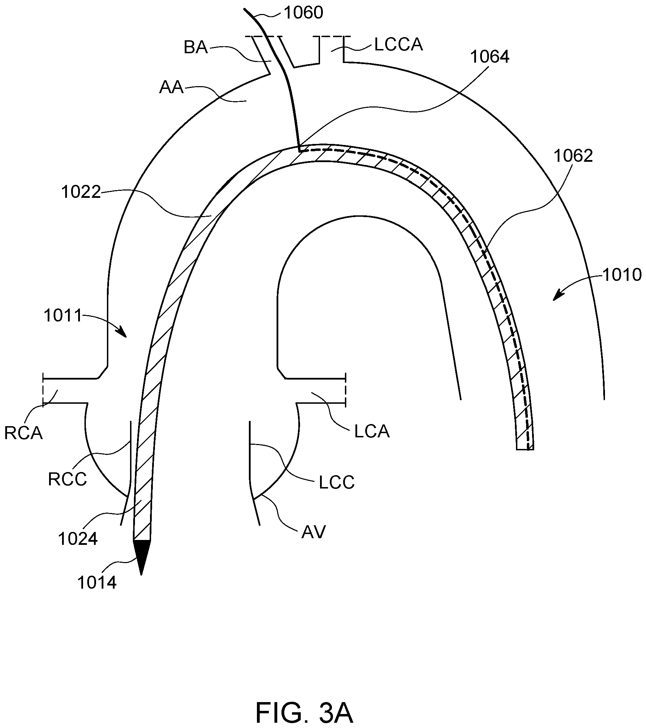

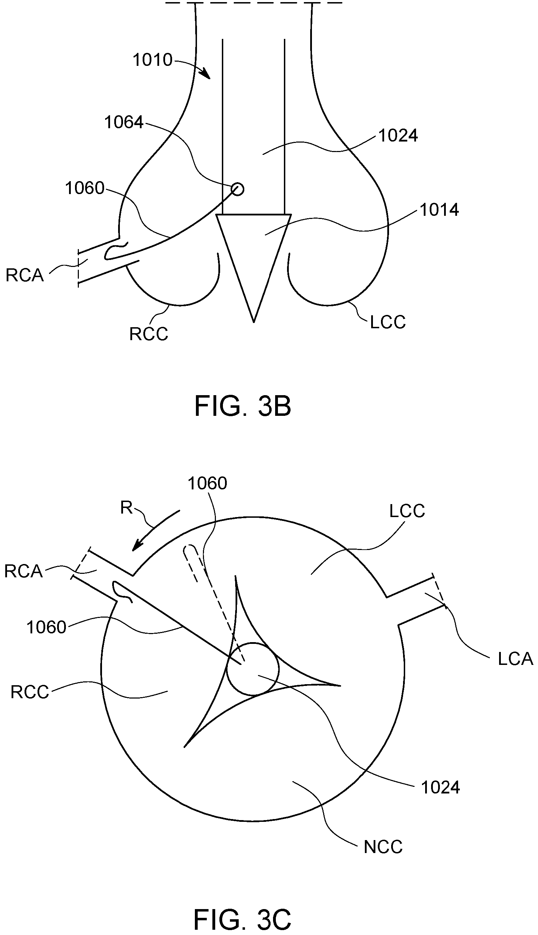

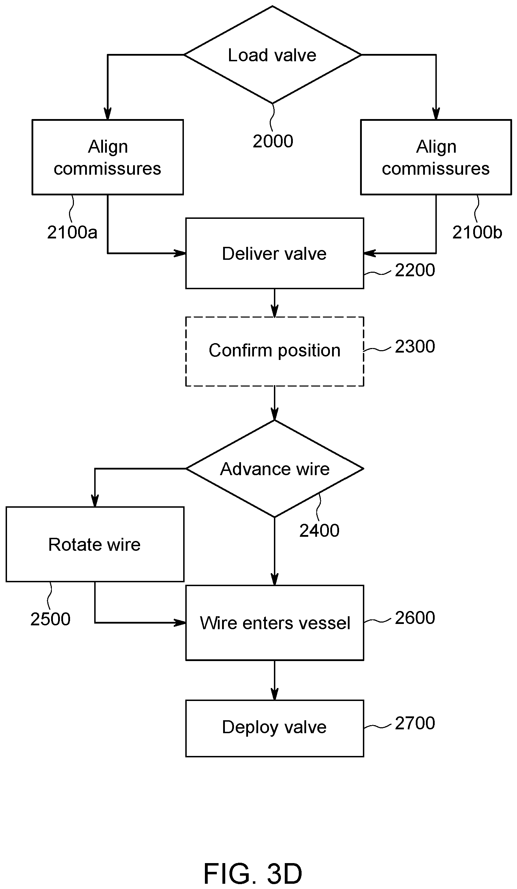

Referring now in addition to Fig. 3A, Fig. 3A illustrates a highly schematic cutaway view of a distal end 1011 of delivery device 1010 after it has been passed in a transfemoral retrograde fashion through a patient, with the distal end portion of the delivery device 1010 being shown traversing the aortic arch AA and with the distal sheath 1024 and distal tip 1014 positioned within the native aortic valve AV. It should be understood that the delivery device 1010 shown in Fig. 3A (as well as that shown in Fig. 3B and Fig. 3C) may be the same or similar to the delivery device 1010 shown in Fig. 2A. However, it should also be understood that the delivery device 1010 shown in Fig. 3A does not need to include all features of the delivery device 1010 shown and described in connection with Fig. 2A, and similarly the delivery device 1010 shown in Fig. 3A may include features in addition to those shown and described in connection with Fig. 2A. The anatomy downstream of the native aortic valve AV may include various branching vessels that may be used to assist in aligning the prosthetic commissures (*e.g.,* commissure features 216 of Fig. 1 and the prosthetic commissures attached thereto) of the prosthetic valve within distal sheath 1024 with the native valve commissures prior to deployment. For example, the first branch of the aortic arch AA is the brachiocephalic artery BA, which supplies blood to the right arm (via the right subclavian artery that branches off from the brachiocephalic artery BA) and to the head and neck (via the right common carotid artery that branches off from the brachiocephalic artery BA). Downstream of the of the brachiocephalic artery BA is the left common carotid artery LCCA, which also supplies blood to the head and neck. Downstream of the left common carotid artery is the left subclavian artery (omitted from Fig. 3) which supplies blood to the left arm. These arteries may be generally referred to herein as aortic head vessels. Immediately downstream of the native aortic valve AV is the right coronary artery RCA, which typically originates above the right coronary cusp RCC, and the left coronary artery LCA, which typically originates above the left coronary cusp LCC. Generally, the right coronary artery RCA and left coronary artery LCA supply blood to heart tissue on the right and left sides of the heart, respectively. There is no third coronary artery corresponding to the non-coronary cusp NCC of the aortic valve AV. These arteries may be referred to herein as the coronary arteries.

The ostia of the aortic head vessels may have a known or pre-defined orientation relative to the native commissures of the aortic valve AV, and that known or pre-defined relative orientation may be substantially consistent among different patients. In other words, if a path is continuously followed along the curvature of the aortic arch AA and to the aortic valve AV, if that path traverses the ostia of the aortic head vessels, upon arriving at the aortic valve AV, the positioning of the distal sheath 1024 relative to the native commissures of the aortic valve AV will be generally predictable. Further, the right coronary artery RCA and left coronary artery LCA are typically aligned with a center portion of the right coronary cusp RCC and the left coronary cusp LCC, respectively. This also means that the right coronary artery RCA and left coronary artery LCA are each typically positioned mid-way (albeit above or downstream) between a circumferentially adjacent pair of native commissures. In particular, as can be seen in Fig. 3C, the right coronary artery RCA is typically positioned mid-way between a first commissure between the non-coronary cusp NCC and the right coronary cusp RCC, and a second commissure between the right coronary cusp RCC and the left coronary cusp LCC. Similarly, the left coronary artery LCA is typically positioned mid-way between the second commissure between the right coronary cusp RCC and the left coronary cusp LCC, and a third commissure between the left coronary cusp LCC and the non-coronary cusp NCC. Although individual anatomies inevitably vary from patient-to-patient, the general relationships described above generally hold true across patient populations, and these relationships may be leveraged to assist with aligning the commissures of the prosthetic heart valve with those of the native aortic valve AV.

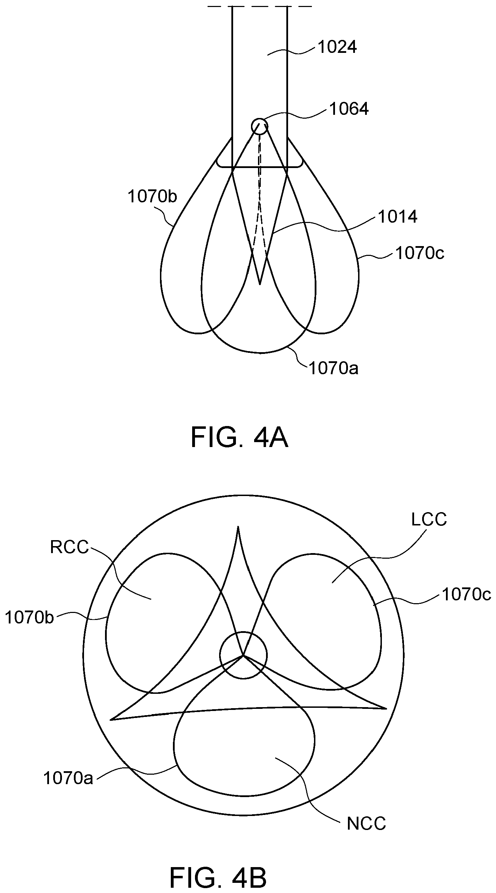

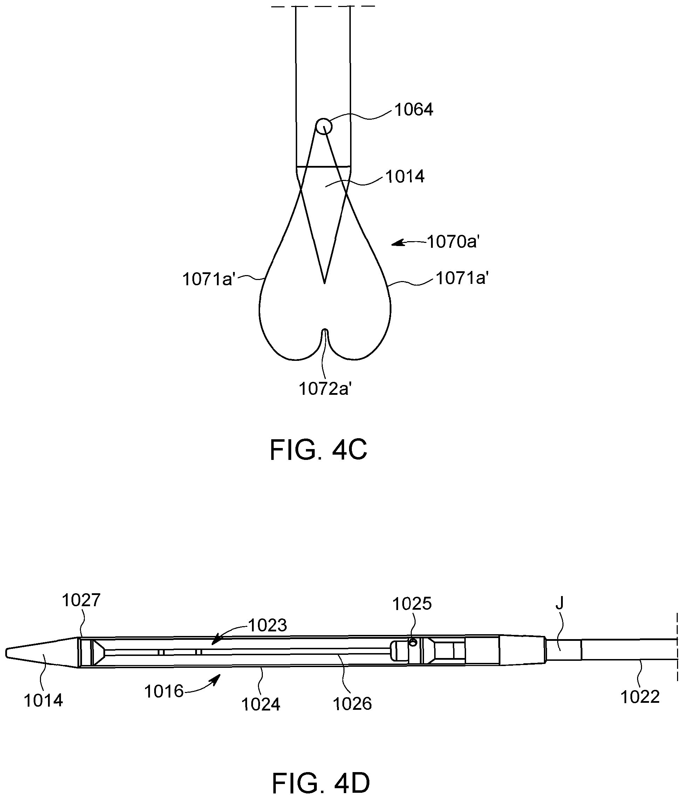

Referring back to Fig. 3A, an example is shown in which the position of the brachiocephalic artery BA is being leveraged to help ensure commissural alignment. In particular, Fig. 3A illustrates an example in which alignment wire 1060 has exited the outer shaft 1022 and has been inserted a distance into the brachiocephalic artery BA. The alignment wire 1060 may be a generally flexible wire, and the alignment wire 1060 may include a blunted or atraumatic distalmost end, which may include for example a 180 degree bend (as shown in Figs. 3B-3C) or pigtail to help ensure the distalmost end of the alignment wire 1060 does not damage tissue it contacts, although other exemplary shapes may be suitable. In some examples, the alignment wire 1060 is flexible enough to remain in a straight condition while within a lumen of the delivery device despite having a set-shape that includes curvature and/or bending. However, it should be understood that the alignment wire 1060 may be provided with a tight loop while in the delivery system prior to being deployed, with the alignment wire 1060 being shape set so that the loop of the alignment wire 1060 can broaden to match the size and/or shape of the relevant anatomy (e.g. the brachiocephalic artery BA). For other embodiments described below, the relevant alignment wires may similarly include a tight loop within the delivery device, with the loop being shape-set to take a shape and/or size relevant to the particular application, such as that of the right coronary artery (Fig. 3B), the nadirs of the leaflets (Fig. 4B), or the commissures of the leaflets (Fig. 4C). According to various examples, the alignment wire 1060 is also stiff enough to be advanced out of the delivery device and remain within a vessel despite forces that may act on the alignment wire. In some examples, the alignment wire 1060 may be formed of a shape-memory material such as a nickel titanium alloy (including nitinol). In some examples, the alignment wire 1060 may be shape-set so that a distal end of the wire is biased to curve, for example at about 90 degrees, as it exits the delivery device 1010. In some examples, the alignment wire 1060 may have a circular cross-section. In other examples, the alignment wire 1060 may have a flat or rectangular cross-section or otherwise have the form of a ribbon. A ribbon-shape alignment wire 1060 may provide certain benefits, including for example better predictability of the alignment wire 1060 taking the set shape as it exits the delivery device 1010.

In one example, the alignment wire may extend through a dedicated lumen 1062 in the wall of the outer shaft 1022. However, in other examples, the alignment wire may be provided in or through other structures of the delivery device 1010. In Fig. 3A, the portion of the alignment wire 1060 still within lumen 1062 is shown in dashed lines. In some examples, the lumen 1062 may terminate in an aperture 1064 that opens to an outer wall of the outer shaft 1022. In some examples, the aperture 1064 is positioned a spaced distance from the distal end of the outer shaft 1022 that corresponds to a general length (or arc length) between the native aortic valve AV and the ostium of the brachiocephalic artery BA. With this configuration, when the distal sheath 1024 is positioned within the annulus of the native aortic valve AV, the aperture 1064 is generally aligned in depth with the ostium of the brachiocephalic artery BA. When the distal sheath 1024 is generally at the desired depth relative to the native aortic valve AV, the user may advance the alignment wire 1060 (which at this point is still mostly or fully within lumen 1062) distally so that it passes through the aperture 1064 to exit the distal shaft 1022. Upon exiting the aperture 1064, the distal end of the alignment wire 1060 may begin to revert to its set shape, for example bending about 90 degrees relative to the longitudinal axis of the delivery device 1010. As advancement of the alignment wire 1060 continues, it is shown in this example entering the brachiocephalic artery BA. A proximal end of alignment wire 1060 may extend proximally to or proximally beyond the handle 1020 to allow for manual movement of the alignment wire 1060, or the alignment wire 1060 may engage with an actuator on the handle 1020 that may be actuated to advance or retract the alignment wire 1060 according to various examples of the disclosure. Although Fig. 3A shows the alignment wire 1060 within a single dedicated lumen 1062 in a wall of the outer shaft 1022, it should be understood that the alignment wire 1060 may be operatively coupled to the delivery device 1010 *(e.g.* to the outer shaft 1022 and/or distal sheath 1024) in other fashions, including within a non-dedicated lumen, within a lumen that is not formed within the wall of the outer shaft 1022, *etc.*

In order to rotationally align the aperture 1064 with the brachiocephalic artery BA, the outer shaft 1022 may be rotated, for example via simple manual rotation of the handle 1020, via actuation of an actuator on the handle 1020 that controls rotation of the outer shaft 1022, or via any other suitable mechanism. According to one example, when the alignment wire 1060 is positioned within the brachiocephalic artery BA, and the distal sheath 1024 is positioned within the native aortic valve AV, the distal sheath 1024 will generally be at a known orientation relative to the native commissures based on the known or pre-defined orientation of the brachiocephalic artery BA relative to the native commissures. Although Fig. 3A shows the distal sheath 1024 closer to the right coronary cusp RCC than the left coronary cusp LCC, in some examples, the alignment wire 1060 has enough stiffness to cause the distal sheath 1024 to be substantially centered within the native aortic valve AV in contrast to the configuration shown in Fig. 3A. In order to ensure commissure alignment upon deployment of the prosthetic heart valve, it may be preferable to load the prosthetic heart valve into the distal sheath 1024 in a specific orientation relative to the angular position of the aperture 1064. For example, the known or pre-defined rotational offset between the ostium of the brachiocephalic artery BA and the native commissures may be used to load the prosthetic heart valve in a desired rotational orientation relative to the aperture 1064 so that, when the delivery device is in the condition shown in Fig. 3A (whether the distal sheath 1024 is centered within the native aortic valve AV or not), the prosthetic commissures are already in rotational alignment with the native commissures. With this configuration, when the distal sheath 1024 is within the native aortic valve AV and the alignment wire 1060 is within the brachiocephalic artery, the three prosthetic leaflets are also rotationally aligned with the three native leaflets. At this point, the distal sheath 1024 may be withdrawn (before, after, or simultaneous to withdrawing the alignment wire 1060 back into the outer shaft 1022) and the prosthetic heart valve may be deployed. Upon deployment, the prosthetic commissures will be aligned with the native commissures since the rotational alignment was achieved prior to the beginning of deployment.

Although Fig. 3A is shown in connection with an alignment wire 1060 positioned within the brachiocephalic artery BA, the alignment wire 1060 may instead be passed into the left common carotid artery LCCA or the right subclavian artery with similar results. However, the brachiocephalic artery BA may be a preferred head vessel to use given that it is the aortic head vessel that is closest to the native aortic valve AV. In some examples, multiple alignment wires 1060 may be provided to cannulate two or more of the aortic head vessels.

Referring now in addition to Fig. 3B, Fig. 3B is a cutaway view of the distal end of the delivery device 1010 positioned within the native aortic valve AV. The main structural difference between the embodiment shown in Fig. 3A and that shown in Figs. 3B-3C is that the aperture 1064 is positioned farther distally (*i.e.,* closer to distal tip 1014), for example in the area of the distal sheath 1024, including near a distal end thereof. The procedure described in connection with Fig. 3A may be generally applicable to the configuration of Figs. 3B-C, except that it is the right coronary artery RCA being cannulated by alignment wire 1060 instead of one of the aortic head vessels. As shown in Fig. 3C, it is possible that when the distal sheath 1024 reaches the area of the native aortic valve AV, the aperture 1064 and/or alignment wire 1060 may not be rotationally aligned with the ostium of the right coronary artery RCA. As explained in connection with Fig. 3A, if this is the case, the outer shaft 1022 and/or distal sheath 1024 may be rotated (*e.g.* manually, via a handle actuator, or any other suitable mechanism) in the rotational direction R until the alignment wire 1060 (shown in dashed lines in the non-aligned position) aligns with the ostium of the right coronary artery RCA according to an example of the disclosure. After the alignment wire 1060 has been advanced into the right coronary artery RCA, the alignment of the delivery sheath 1024 relative to the native commissures and native leaflets will be known. As described in connection with Fig. 3A, the prosthetic heart valve may be loaded into the delivery sheath 1024 in a known orientation (*e.g.* with one of the prosthetic leaflets centered with respect to aperture 1064) so that, upon deployment of the prosthetic heart valve, one of the prosthetic leaflets is rotationally aligned with the right coronary cusp RCC. As long as one prosthetic leaflet is rotationally aligned with one of the native leaflets, all of the prosthetic leaflets and commissures will be rotationally aligned with corresponding native leaflets and commissures. Further, as described in connection with Fig. 3A, the alignment wire 1060 may be withdrawn back into the distal sheath 1024 either before, after, or during retraction of the distal sheath 1024 to deploy the prosthetic heart valve.

Although Figs. 3B-3C illustrate the right coronary artery RCA being cannulated with the alignment wire 1060, in other embodiments the left coronary artery LCA may instead be cannulated with similar results. The right coronary artery RCA may be preferred in some embodiments, for example, because delivery devices (particularly those without deflection control) may tend to be positioned closer to the right coronary artery RCA than the left coronary artery LCA when the prosthetic heart valve is at the desired depth relative to the native aortic valve AV. A stiff alignment wire 1060 may help "push" the distal sheath 1024 away from the right coronary artery RCA to help center the distal sheath 1024 within the native aortic valve AV. However, in some examples, it may be preferable to cannulate the left coronary artery LCA if the distal sheath 1024 is positioned closer to the right coronary artery RCA, for example because there may be more space between the distal sheath 1024 and the left coronary artery LCA which may make it easier to steer the alignment wire from the aperture 1064 into the left coronary artery LCA. In some examples, two alignment wires may be provided so that both the right coronary artery RCA and left coronary artery LCA may be cannulated for ensuring commissure alignment. Still in further examples, two or more alignment wires may be provided so that one or more coronary arteries may be cannulated while simultaneously one or more aortic head vessels are cannulated. Still further, although Figs. 3A-3C are shown and described in connection with a delivery device (e.g., delivery device 1010) that is suited for self-expanding valves (e.g., prosthetic heart valve 200), the same concepts may be applied to other delivery devices, including delivery devices (*e.g.* balloon catheters) configured to deploy balloon-expandable prosthetic heart valve or configured to deploy mechanically expandable prosthetic heart valves. In these embodiments, the prosthetic heart valves would also be pre-loaded into or onto the corresponding delivery device in a known rotational orientation relative to one or more of the alignment wire(s) 1060 and/or apertures 1064 so that the orientation of these vessels relative to the native aortic valve may be leveraged to help ensure commissure alignment.

Examples of methods of use of configurations of the type shown and described in connection with Figs. 3A-C are shown in the flow chart of Fig. 3D. For example, at a first step 2000, the prosthetic heart valve (*e.g.,* prosthetic heart valve 200) may be loaded into a delivery device (*e.g.,* the compartment 1023 of delivery device 1010). If the configuration of Fig. 3A (or a similar configuration) is being used, in step 2100a, the prosthetic heart valve may be loaded so that the prosthetic commissures (*e.g.,* the portions of prosthetic leaflets 208 coupled to the commissure features 216 in Fig. 1) are rotationally offset relative to aperture 1064 by about the same rotational offset (*e.g.* +/- 5 degrees) as the rotational offset between the relevant aortic head vessel *(e.g.* brachiocephalic artery BA) and the native commissures. If the configuration of Figs. 3B-3C (or a similar configuration) is being used, in step 2100b, the prosthetic heart valve may be loaded so that the prosthetic commissures are rotationally offset relative to aperture 1064 by about the same rotational offset (*e.g.* +/- 5 degrees) as the rotational offset between the relevant coronary artery (*e.g.* right coronary artery RCA) and the native commissures. With the prosthetic heart valve loaded into the delivery device, the delivery device may be advanced (*e.g.,* via a retrograde transfemoral route) until the collapsed prosthetic heart valve is positioned within or adjacent to the native aortic valve AV in step 2200. In optional step 2300, desired positioning (*e.g.* axial and/or rotational) of the prosthetic heart valve (or component(s) of the delivery device) relative to the native aortic valve AV (or relative to other anatomical landmarks) may be confirmed via any suitable imaging modality such as fluoroscopy. It should be understood that optional step 2300 may be performed at different or additional times during the process. In step 2400, the alignment wire 1060 may be advanced so that it exits the delivery device through the aperture 1064. If needed or desired, at step 2500, the delivery device may be rotated to help align the alignment wire 1060 with the ostium of the relevant vessel (*e.g.* the brachiocephalic artery BA or the left coronary artery LCA), which may be performed under imaging if desired (*e.g.,* similar to as shown in Fig. 3C). Whether or not step 2500 is performed, the alignment wire 1060 may be advanced further distally in step 2600 until the alignment wire 1060 enters a distance into the relevant vessel. Whether the alignment wire 1060 is left within the relevant vessel, or retracted back into the delivery device having confirmed the desired alignment, the prosthetic heart valve may be deployed into the native aortic valve AV in step 2700 (*e.g.,* by retracting distal sheath 1024 to allow the prosthetic heart valve to self-expand). Upon deployment, the prosthetic commissures of the prosthetic heart valve will be rotationally aligned with the native commissures of the prosthetic aortic valve AV as a result of the prior alignment steps.

Although some of the embodiments described above are generally directed to leveraging positions of the vessel branches relative to the native aortic valve to assist with commissure alignment and/or centering of the delivery device within the native aortic valve, in other embodiments, the aortic and/or mitral valve apparatus may instead (or additionally) be leveraged to assist with commissure alignment and/or centering.

Referring now in addition to Fig. 4A, Fig. 4A is side view of distal end of delivery device 1010 with three alignment wires 1070a-1070c in a deployed configuration. It should be understood that the delivery device 1010 shown in Fig. 4A (as well as that shown in Fig. 4B and Fig. 4C) may be the same or similar to the delivery device 1010 shown in Fig. 2A. However, it should also be understood that the delivery device 1010 shown in Figs. 4A-4C does not need to include all features of the delivery device 1010 shown and described in connection with Fig. 2A, and similarly the delivery device 1010 shown in Figs. 4A-4C may include features in addition to those shown and described in connection with Fig. 2A. Similar to the configuration of Fig. 3B, the distal sheath 1024 may include an aperture 1064 near a distal end thereof. In the specific example of Figs. 4A-B, multiple apertures, such as three apertures, may be provided, preferably at substantially equal intervals around the circumference of the distal sheath 1024 (*e.g.,* every 120 degrees, +/- 5 degrees). The delivery device 1010 may include three alignment wires 1070a-1070c, one corresponding to each aperture 1064, although in other embodiments, more or fewer alignment wires may be provided (*e.g.,* two alignment wires for treatment of a bi-leaflet valve). Each alignment wire 1070a-1070c may in some examples be radiopaque. In the illustrated example, each alignment wire 1070a-c has a looped distal end, with a proximal end extending proximally through a lumen of the delivery device 1010.

In various examples, during delivery of a prosthetic heart valve mounted to the delivery device 1010 (*e.g.,* maintained within distal sheath 1024 in a collapsed condition), the alignment wires 1070a-1070c may be partially or fully retracted within the corresponding apertures 1064 so that they do not interfere with tissue during advancement of the delivery device 1010 through the vasculature. Once the distal tip 1014 of the delivery device 1010 is positioned at or near the native aortic valve AV (*e.g.,* following retrograde, transfemoral delivery), the alignment wires 1070a-1070c may be advanced so that they exit via the apertures 1064 according to one example. As the alignment wires 1070a-1070c exit the apertures 1064, for the embodiment shown in Figs. 4A-4B, the alignment wires 1070a-1070c may form looped ends. The loops may be preset shapes, for example, if the alignment wires 1070a-1070c are formed a shape memory material, such as nickel-titanium alloys, such as nitinol. However, in other examples, the alignment wires 1070a-1070c are not formed of shape memory materials, but still tend to take the loop shape as they become free of their confinement within respective lumen(s) within the delivery device 1010.

In some examples, delivery device 1010 may be capable of active rotation, similar to as described above in connection with Fig. 3C. After the alignment wires 1070a-1070c are advanced and form the example loop shape shown in Fig. 4A, the delivery device 1010 may be rotated until the loops generally align with the nadirs of the three native leaflets, which may be confirmed under imaging such as fluoroscopy if the alignment wires are radiopaque. The delivery device 1010 may be advanced until the loops of the alignment wires 1070a-1070c contact the nadirs of the native leaflets, as shown in Fig. 4B, which may serve as confirmation that the delivery device 1010 is in a known rotational orientation relative to the native heart valve. As with the embodiment described in connection with Figs. 3A-3C, the prosthetic heart valve may be pre-loaded onto or into the delivery device 1010 with a known rotational orientation relative to the delivery device. For example, referring back to Fig. 4A, the prosthetic heart valve (e.g., prosthetic heart valve 200 of Fig. 1) may be loaded into a collapsed condition within the distal sheath 1024 with the circumferential center of each prosthetic leaflet rotationally centered along one of the three apertures 1062. With this configuration, when the alignment wires 1070a-1070c are in contact with the nadirs of the native leaflets as shown in Fig. 4B, each prosthetic commissure will be rotationally aligned with a corresponding native commissure, and each prosthetic leaflet will be rotationally aligned with a corresponding native leaflet. It should also be understood that, when the alignment wires 1070a-1070c are in contact with the nadirs of the native leaflets as shown in Fig. 4B, the contact may tend to force the distal sheath 1024 toward the radial center of the native aortic valve AV.

Once the desired rotational orientation is confirmed via fluoroscopy or otherwise, the prosthetic heart valve may be deployed into the native aortic valve annulus. In some examples, it may be preferable to deploy the prosthetic heart valve while the alignment wires 1070a-1070c remain in contact with the nadirs of the native leaflets. However, in other examples, the alignment wires 1070a-1070c may become obstacles to the deployment of the prosthetic heart valve. Thus, in some examples, prior to (or simultaneous to) deploying the prosthetic heart valve, the alignment wires 1070a-1070c may be retracted back into the delivery device. As with alignment wire 1060, alignment wires 1070a-1070c may extend proximally to or proximally beyond handle 1020 so that the alignment wires may be manually manipulated or manipulated via an actuator on the handle 1020 to which the alignment wires 1070a-1070c are attached.

Although Figs. 4A-4B are described in the context of actively rotating the delivery device 1010 to align the alignment wires 1070a-1070c with the nadirs of the native leaflets, in other embodiments the delivery device (e.g., modifications of the delivery device 1010 disclosed above) may be capable of passive rotation. Passive rotation may be accomplished via a joint bearing J (*see, e.g.,* Fig. 4D), such as a rotary bearing *(e.g.* a thrust bearing). In one example, a thrust bearing may connect the distal shaft 1022 to the distal sheath 1024 so that the distal sheath 1024 is at least partially freely rotatable about its longitudinal axis. A similar bearing may be provided on the inner shaft 1026, for example just proximal to the proximal hub or retainer 1025. Whether one joint or multiple joints provide for the ability for the prosthetic heart valve to rotate while maintained within compartment 1023, as the alignment wires 1070a-1070c are advanced and take their loop form, and as the delivery device is advanced to contact the alignment wires 1070a-1070c with the native leaflets, the loops of the alignment wires 1070a-1070c may naturally tend to slide along the native leaflets until they reach the nadirs. As this sliding occurs, the distal sheath 1024 may passively rotate via the joint(s) J. If passive rotation is used, it may be desirable to provide a rotation lock for the j oint J. For example, after the desired alignment is achieved, prior to withdrawing the alignment wires 1070a-1070c into the delivery device 1010 in preparation for deployment of the prosthetic heart valve, the rotation lock may be activated (for example by tensioning or compressing the bearing(s) to friction lock them against further rotation) so that the prosthetic heart valve does not lose rotational alignment via unintentional passive rotation of the distal sheath 1024 during deployment. This type of rotational lock may also be used if the delivery device is actively rotated, although such a rotational lock may not be needed in a system that allows for active rotation of the prosthetic heart valve.

While the alignment wires 1070a-1070c may form loops as shown in Figs. 4A-4B to assist with positioning the alignment wires 1070a-1070c within the nadirs of the native leaflets, in other examples, the native leaflet commissures may be targeted instead of the native leaflet nadirs. For example, Fig. 4C illustrates a configuration that is identical to that of Fig. 4A, except that the alignment wires have a different shape. Although only one alignment wire 1070a' and one corresponding aperture 1064 is shown in Fig. 4C, it should be understood that three alignment wires 1070a' and three corresponding apertures 1064 may be provided in the case of replacing a native aortic valve or other three-leaflet valve. When the alignment wire 1070a' is advanced, it forms a general loop shape (including, *e.g.,* a heart shape or a cardioid shape) that may include two wire portions 1071a' that extend from the aperture 1064 distally and which begin to separate from each other. However, at the distal end of the alignment wire 1070a', the two wire portions 1071a' may each loop or hook proximally and meet at an apex. The proximally hooking wire portions 1071a' may form a recess 1072a' that is sized and shaped to hook over a native leaflet commissure, with the distal-most portions of the two wire portions 1070a' contacting the native leaflets on either side of the native commissure. With this embodiment, when the three alignment wires 1070a' are advanced, they may be actively or passively rotated until the three recesses 1072a' align with the three corresponding native commissure. In one example, when the alignment wires 1070a' engage the three native commissures, the configuration looks like that shown in Fig. 4B, except that the alignment wires are shifted about 60 degrees to align with the native commissures instead of the native leaflet nadirs. It should also be understood that, for embodiments that include alignment wires that engage the native commissures, the prosthetic valve may need to be pre-loaded in a different orientation. For example, for the embodiment shown in Fig. 4C, the prosthetic heart valve would preferably be pre-loaded onto the delivery device so that the commissures of the prosthetic heart valve rotationally align with the three apertures 1064.

Although the alignment members shown and described in connection with Figs. 4A-4C are described as alignment "wires," it should be understood that other structures beyond wires may be suitable for assisting with alignment in a similar fashion. For example, in some embodiments the alignment members may be wires, but not form looped ends, but rather simply straight ends *(e.g.* a single non-looped wire forming a straight arm) or other more intricate shapes. In some examples, the alignment wires 1070a-1070c are inflatable. Other non-wire structures, such as inflatable balloon members, may also be used in place of alignment wires 1070a-1070c with otherwise similar or identical functionality. Still further, although generally described in the context of aortic valve replacements, the embodiments described in connection with Figs. 4A-4C may be suitable for use in replacing other valves, including the pulmonary valve, mitral valve, or tricuspid valve. Modifications, such as matching the total number and relative positioning of the alignment wires to the number and positioning of the native leaflets, may be made if desired when treating the other valves. In other words, a system similar to those shown in Figs. 4A-4C may be used to replace the bi-leaflet mitral valve, and the system may be modified to include two alignment wires generally 180 degrees apart (+/- 5 degrees) to match the configuration of the native mitral valve.

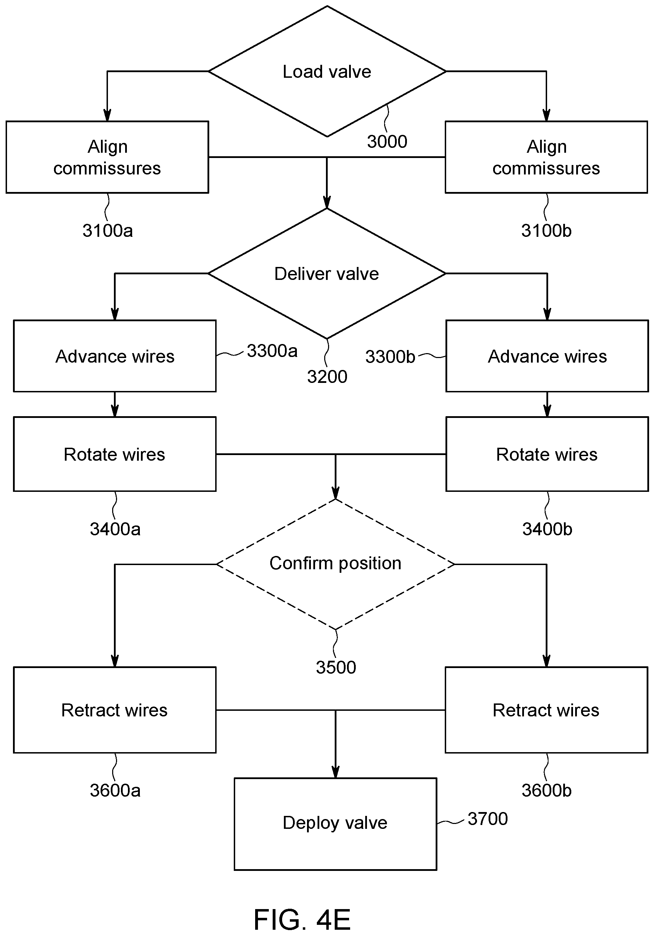

Examples of methods of use of configurations of the type shown and described in connection with Figs. 4A-D are shown in the flow chart of Fig. 4E. For example, at a first step 3000, the prosthetic heart valve (*e.g.,* prosthetic heart valve 200) may be loaded into a delivery device (*e.g.,* the compartment 1023 of delivery device 1010). If the configuration of Figs. 4A-B (or a similar configuration) is being used, in step 3 100a, the prosthetic heart valve may be loaded so that the prosthetic commissures (*e.g.,* the portions of prosthetic leaflets 208 coupled to the commissure features 216 in Fig. 1) are rotationally offset relative to apertures 1064 so that the prosthetic commissure are aligned (+/- 5 degrees) with a circumferential center between two adjacent ones of the apertures 1064. If the configuration of Fig. 4C (or a similar configuration) is being used, in step 3100b, the prosthetic heart valve may be loaded so that the prosthetic commissures are rotationally aligned (+/- 5 degrees) with the apertures 1064. With the prosthetic heart valve loaded into the delivery device, the delivery device may be advanced (*e.g.,* via a retrograde transfemoral route) until the collapsed prosthetic heart valve is positioned adjacent to the native aortic valve AV in step 3200. At this point (or at a time just prior to or just after), the user may advance the alignment wires 1070a-1070c distally out of apertures 1064 in step 3300a (or advance the alignment wires 1070a' out of apertures 1064 in step 3300b for the configuration of Fig. 4C). Prior to or during contacting the alignment wires 1070a-1070c (or alignment wires 1070a') with the native leaflets, the alignment wires 1070a-1070c may be actively or passively rotated so that they sit within the nadirs of the native leaflets in step 3400a, or the alignment wires 1070a' may be actively or passively rotated so that the recesses 1072a' overlie the commissures of the native leaflets in step 3400b. In optional step 3500, desired positioning (*e.g.* axial and/or rotational) of the prosthetic heart valve (or component(s) of the delivery device) relative to the native aortic valve AV (or relative to other anatomical landmarks) may be confirmed via any suitable imaging modality such as fluoroscopy. It should be understood that optional step 3500 may be performed at different or additional times during the process. After the prosthetic heart valve is in the desired orientation relative to the native aortic valve AV, the alignment wires 1070a-1070c may be retracted back through the apertures 1064 in step 3600a (or the alignment wires 1070a' may be retracted through the apertures 1064 in step 3600b). Then, in step 3700, the prosthetic heart valve may be deployed into the native aortic valve AV (*e.g.,* by retracting distal sheath 1024 to allow the prosthetic heart valve to self-expand). Upon deployment, the prosthetic commissures of the prosthetic heart valve will be rotationally aligned with the native commissures of the prosthetic aortic valve AV as a result of the prior alignment steps.

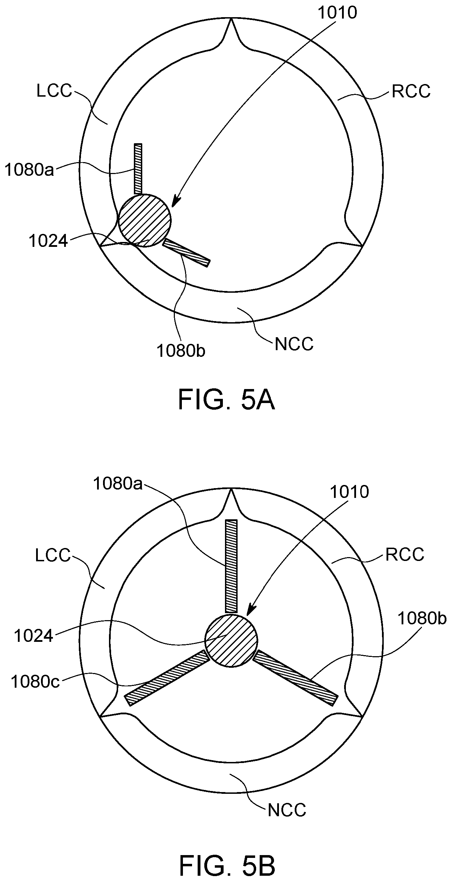

Referring now in addition to Figs. 5A-B, Figs. 5A-B are cutaway views of examples of the capsule or distal sheath 1024 of delivery device 1010 positioned within the native aortic valve while the valve is opened (*i.e.* while the three leaflets are not coapted). It should be understood that the delivery device 1010 shown in Fig. 5A (as well as that shown in Fig. 5B) may be the same or similar to the delivery device 1010 shown in Fig. 2A. However, it should also be understood that the delivery device 1010 shown in Fig. 5A does not need to include all features of the delivery device 1010 shown and described in connection with Fig. 2A, and similarly the delivery device 1010 shown in Fig. 5A may include features in addition to those shown and described in connection with Fig. 2A. One or more fins 1080a-1080c may extend radially outwardly from the distal sheath 1024. In Fig. 5A, two fins 1080a-1080b are provided at about 120 degrees (+/- 5 degrees) apart from each other (or 240 degrees (+/- 5 degrees) depending on the direction of measurement). In the example of Fig. 5B, three fins 1080a-1080c are provided, with each pair of adjacent fins being separated by about 120 degrees. It should be understood that, in other embodiments, a single fin 1080a may be provided. When used in a native valve having three leaflets *(e.g.* the aortic valve or pulmonary valve), one, two, or three fins may be desirable, but when used in a native valve having two leaflets *(e.g.* the mitral valve), one or two fins may be desired. If more than one fin is provided, it may be preferable (but not necessary) for each fin to be identical in structure to each other.

According to various examples, the one or more fins 1080a-1080c may be formed as thin members, either integral with or mounted to the distal sheath 1024. In some examples, each fin 1080a-1080c may have a thickness that is smaller than the diameter of the distal sheath 1024. In some examples, each fin 1080a-1080c has a width that is no greater than about half the diameter of the native aortic valve. It should be understood that, as used herein, width refers to the dimension shown in Figs. 5A-5B extending radially away from the distal sheath 1024, whereas thickness refers to the other dimension shown in Figs. 5A-5B for fins 1080a-1080c. In some situations, the width of the fins 1080a-1080c is less than half of the diameter of the native aortic valve so that, if the terminal ends of the fins 1080a-1080c nest within the native commissures (as shown Fig. 5B), there is enough space for each fin 1080a-1080c within the boundaries the native aortic valve. For example, the width of the fins 1080a-1080c may be large enough so that the fins 1080a-1080c can directly nest within the native commissures, as shown in Fig. 5B. However, there is no set lower limit on the width of the fins 1080a-1080c. For example, the fins 1080a-1080b shown in Fig. 5A have a smaller width than the fins 1080a-1080c shown in Fig. 5B, and instead of nesting directly within the native commissures, the fins 1080a-1080c may be sandwiched between pairs of leaflets upon leaflet coaptation. As is described in greater detail below, in some examples, the width of each fin 1080a-1080c is enough that, as the native leaflets close, the native leaflets are able to readily contact the fins 1080a-1080c and impart force on the fins 1080a-1080c to assist with alignment. The length of the fins 1080a-1080c, although not shown in Figs. 5A-B, is measured along the length axis of the delivery device 1010 and, in some examples, is not greater than the length of the distal sheath 1024. In some examples, the length of the fins 1080a-1080c is substantial enough to provide suitable contact area between the native leaflets and the fins 1080a-1080c as the native leaflets close onto or over the fins. Although the term "fins" is used in connection with Figs. 5A-5B, it should be understood that the term fins is not intended to be limiting, and instead the features may be tabs, wings, or any other structures that perform the functions described in connection with Figs. 5A-5B.

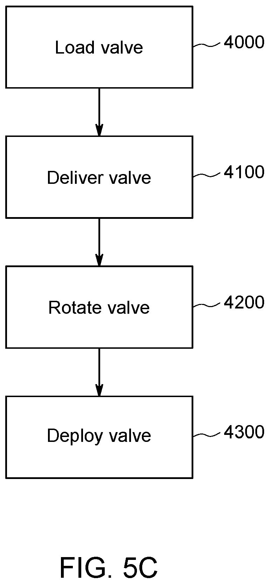

In one example of use, as shown in the flow chart of Fig. 5C, a prosthetic heart valve (such as prosthetic heart valve 200) may be loaded into the capsule or distal sheath 1024 so that one of the prosthetic commissures rotationally aligns with a corresponding one of the fins 1080a-1080c, as represented by step 4000. If more than one fin is provided, as long as one prosthetic commissure is rotationally aligned with one fin, all remaining fins will be aligned with a commissure of the prosthetic heart valve. After loading the prosthetic heart valve into the distal sheath 1024, the delivery device 1010 may be advanced into the patient until the distal sheath 1024, including the one or more fins 1080a-c, are axially aligned with the native heart valve *(e.g.* in the depth direction), as represented by step 4100. The native heart valve may be the aortic valve, the mitral valve, the pulmonary valve, or the tricuspid valve. In some examples, the distal sheath 1024 is rotatably joined to the outer shaft 1022 *(e.g.* via a rotary bearing such as a thrust bearing similar to the schematic shown in Fig. 4D) so that, as the prosthetic leaflets coapt into contact with the one or more fins 1080a-1080c, the force applied to the fins 1080a-1080c by the closing leaflets causes the distal sheath 1024 to rotate until each fin is positioned between two adjacent closed leaflets, as represented by step 4200. In one example, the inner shaft 1026 may also be provided with a rotary bearing, for example just proximal to the proximal hub and/or retainer 1025, so that the inner shaft 1026 may rotate along with the distal sheath 1024. Fig. 5A illustrates an exemplary position of the distal sheath 1024 and fins 1080a-1080b prior to the native leaflets closing. As the leaflets close, they will tend to not only rotate the fins 1080a-1080b into alignment between pairs of closing leaflets, but may also push the distal sheath 1024 toward the radial center of the native heart valve. When the one or more fins 1080a-1080c are aligned between pairs of closed leaflets, the fins 1080a-1080c are also aligned with the native commissures (as shown in Fig. 5B). Due to the original positioning of the prosthetic heart valve within the distal sheath 1024 during loading, when the fins 1080a-1080c are aligned with the native commissures, the commissures of the prosthetic heart valve are also aligned with the native commissures. Thus, once this alignment is achieved, the prosthetic heart valve may be deployed from the delivery device 1010, as represented by step 4300, for example by withdrawing the distal sheath 1024 relative to the prosthetic heart valve to allow the prosthetic heart valve to expand into the native aortic valve. Upon expansion, the prosthetic commissures will be rotationally aligned with the native commissures. In some examples described above, this rotational alignment may be passive in the sense that the force of the leaflets on the fins 1080a-1080c is the only force causing the distal sheath 1024 to rotate to move the fins 1080a-1080c into rotational alignment with the native commissures. In some examples, active alignment may be used (either in combination with, or as an alternate to passive alignment) to actively rotate the distal sheath 1024 and associated one or more fins 1080a-1080c. Mechanisms to allow for active and/or passive rotation of the distal sheath 1024 may include any suitable mechanisms, including those described in connection with other embodiments herein.

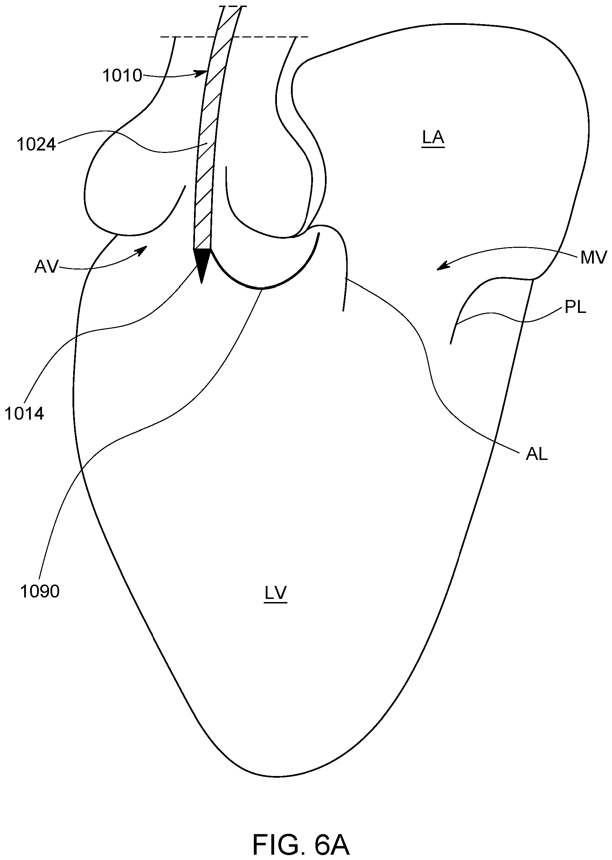

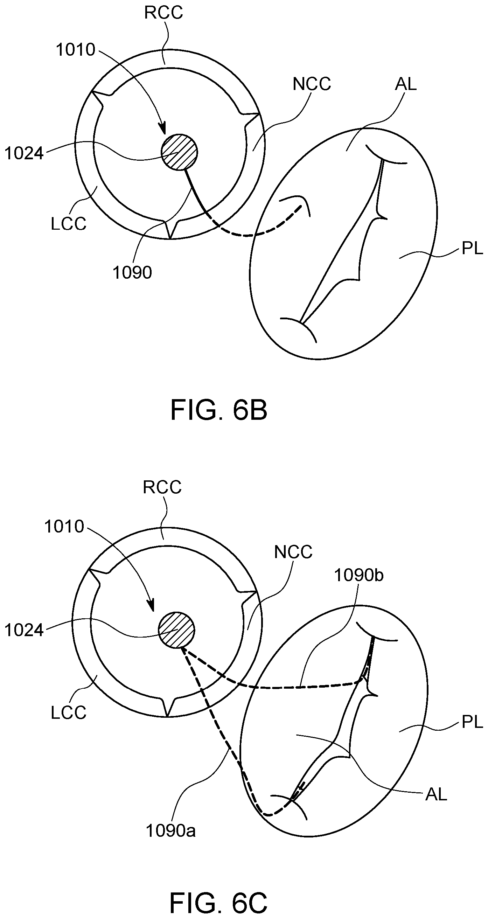

Referring now in addition not Fig. 6A, Fig. 6A is a highly schematic cutaway side view of the left heart with a delivery device 1010 positioned within an aortic valve AV. It should be understood that the delivery device 1010 shown in Fig. 6A (as well as that shown in Figs. 6B-6D) may be the same or similar to the delivery device 1010 shown in Fig. 2A. However, it should also be understood that the delivery device 1010 shown in Figs. 6A-6D does not need to include all features of the delivery device 1010 shown and described in connection with Fig. 2A, and similarly the delivery device 1010 shown in Figs. 6A-6D may include features in addition to those shown and described in connection with Fig. 2A. The mitral valve MV, which separates the left atrium LA from the left ventricle LV, is illustrated in Fig. 6A with its anterior leaflet AL and posterior leaflet PL in the open condition, and the leaflets of the aortic valve AV closed over the distal end of a delivery device, which in this example may be delivery device 1010. The embodiments described in connection with Figs. 6A-6D utilize interaction of the delivery device 1010 (or a related component) with the mitral valve MV to help achieve commissure alignment and/or centering of the delivery device 1010 within the native aortic valve AV. For example, there may be a known or pre-defined relative orientation between (i) the center of the anterior leaflet AL of the mitral valve and either the center of one of the aortic valve AV leaflets or aortic valve commissures or between (ii) the mitral valve commissures and either the center of one of the aortic valve AV leaflets or aortic valve commissures. That known or pre-defined relative orientation may be utilized in a way similar to that described in connection with Figs. 3A-4B to assist with commissure alignment. For example, Figs. 6B-6C illustrate a highly schematic top view of the aortic valve AV and its position next to the mitral valve MV with other structures of the heart omitted for clarity. Very generally, the center of the anterior leaflet AL of the mitral valve MV may generally align with a center of the non-coronary cusp NCC, with the commissure between the non-coronary cusp NCC and left coronary cusp LCC, or a location therebetween. However, it should be understood that if a different pre-defined relationship exists between the orientation of the mitral valve and that of the aortic valve, that pre-defined relationship may be leveraged for achieving the desired orientation according to additional examples of the disclosure.

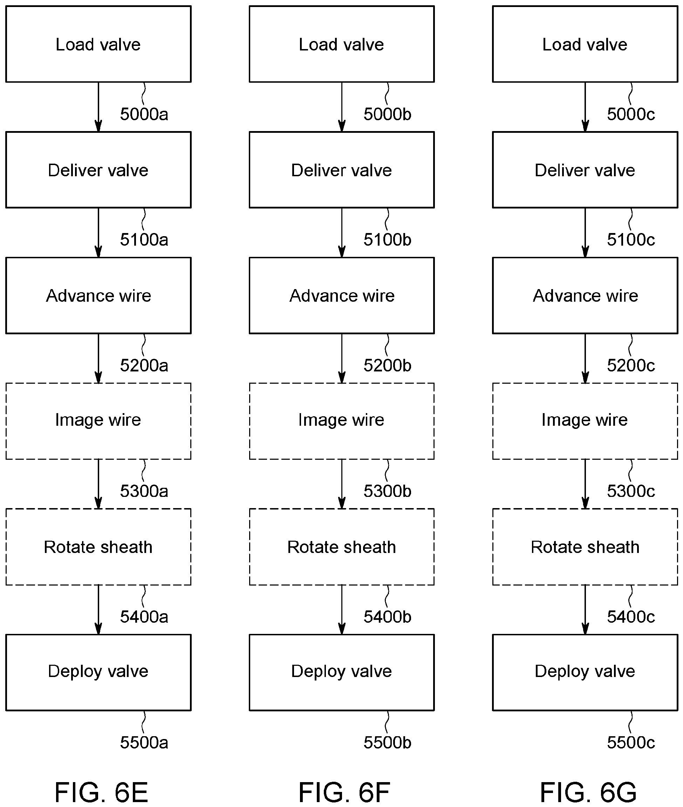

In one example of using this pre-defined relationship, as shown in Figs. 6A-6B, an alignment wire 1090 may be advanced from the delivery device 1010, for example from distal sheath 1024, extending downwardly into the left ventricle LV, and hooking back upwardly until it contacts a center of the outflow surface or underside of the anterior leaflet AL of the mitral valve MV. Referring in addition to Fig. 6B, the portion of the alignment wire 1090 visible in the picture is shown in solid lines, while the portion hooking up back toward the outflow surface of the anterior leaflet AL is shown in dashed lines. The alignment wire 1090 may be similar or identical to alignment wire 1060 in shape and structure, and is thus not described again here. The distal sheath 1024 may have an aperture 1064 similar or identical to that shown and described in connection with Fig. 3B, although other configurations of alignment wire 1090 and aperture 1064 may be suitable, for example. In an exemplary method of use, as represented in the flow chart of Fig. 6E, the prosthetic heart valve mounted to delivery device 1010 may be mounted, in step 5000a, in a rotational orientation so that the center of one of the prosthetic leaflets is generally oriented in alignment with the aperture 1064 from which the alignment wire 1090 will extend. However, as noted above, if the pre-defined relationship between the mitral valve MV and the aortic valve AV is different, the prosthetic heart valve may be loaded into the delivery device in step 5000a with a correspondingly different orientation. In step 5100a, after loading the prosthetic heart valve into or onto the delivery device 1010 in the desired orientation, the delivery device 1010 may be advanced toward the aortic valve AV, for example via a retrograde transfemoral route, until the distal sheath 1024 is near or within the native aortic valve AV. Before this point, the alignment wire 1090 may be maintained within a lumen of the delivery device 1010, for example within a lumen in a wall of the distal sheath 1024. After this point, as represented by step 5200a, the alignment wire 1090 may be advanced to exit the distal sheath 1024, at which point the alignment wire 1090 may begin to revert to its set shape and curve in a desired direction back upwardly toward the center of the outflow surface of the anterior leaflet AL. In some examples, as represented by optional step 5300a, the position of the alignment wire 1090 is viewed under imaging, for example under fluoroscopy if the alignment wire 1090 is radiopaque. Further, the distal sheath 1024 may be actively rotated, as represented by optional step 5400a, to help align and guide the alignment wire 1090 until it contacts the center of the anterior leaflet AL, preferably at a base thereof, as best shown in Fig. 6B. Once the desired contact is confirmed, for example under visualization, the commissures of the prosthetic heart valve will already be aligned with the commissures of the native aortic valve AV. For example, if the pre-defined relationship is that the center of the anterior leaflet AL is aligned with the center of the non-coronary cusp NCC, and the prosthetic heart valve is loaded into the distal sheath 1024 with the center of one of the leaflets aligned with the aperture 1064, when the configuration of Fig. 6B is achieved, the center of one of the prosthetic leaflets is aligned with the center of the non-coronary cusp NCC. As noted elsewhere, as long as one prosthetic leaflet (or one prosthetic commissure) is in rotational alignment with one native leaflet (or one native commissure), all of the prosthetic commissures will be aligned with all of the native commissures. However, if the pre-defined relationship is other than the center of the anterior leaflet AL aligning with the center of the non-coronary cusp NCC, that pre-defined relationship may be utilized to achieve commissure alignment in the same way. In addition to assisting with achieving commissure alignment, the force of the alignment wire 1090 on the anterior leaflet AL may cause the distal sheath 1024 to push toward the center of the native aortic valve AV, helping to achieve desirable centering prior to deployment of the prosthetic heart valve. Once the desired positioning and/or centering is achieved, as represented by step 5500a, the prosthetic heart valve may be deployed, for example by retracting the distal sheath 1024 relative to the prosthetic heart valve, either before, after, or simultaneous with retracting the alignment wire 1090 back into the distal sheath 1024.

While using a single alignment wire 1090 to contact the base and/or center of the anterior leaflet AL may provide for helpful commissure alignment and centering of the prosthetic heart valve, in another example, two alignment wires 1090a-1090b may be used to latch onto or otherwise contact the commissures of the native mitral valve MV, as shown in the example of Fig. 6C. One benefit of using two alignment wires 1090a-1090b compared to a single alignment wire 1090 may be that two points of contact provide for increased stability of the position of the distal sheath 1024 while the alignment wires 1090a-1090b are latched onto or otherwise in contact with the native mitral valve MV. Each alignment wire 1090a-1090b may be similar or identical to alignment wire 1090 in structure. Each alignment wire 1090a-b may have a dedicated lumen and associated aperture in delivery device 1010, but in other examples the alignments wires 1090a-1090b may share a lumen and aperture. In one example, the alignment wires 1090a-1090b are shape-set so that, as they exit their associated lumen and are advanced away distally from the distal sheath 1024, they extend downwardly and then begin to hook back up toward the native mitral valve MV, curving in different directions so that one alignment wire 1090a can be positioned on, nested in, or latched onto one of the two native mitral valve commissures, while the other alignment wire 1090b can be positioned on, nested in, or latched onto the other of the two native mitral valve commissures. As with the single alignment wire 1090 embodiment, the prosthetic heart valve may be mounted in or on the delivery device 1010 in a pre-determined rotational orientation (as represented by step 5000b in the flow chart of Fig. 6F) so that, when the alignment wires 1090a-1090b are nested in the mitral valve commissures, the commissures of the prosthetic heart valve are in rotational alignment with the commissures of the native aortic valve AV. In step 5100b, after loading the prosthetic heart valve into or onto the delivery device 1010 in the desired orientation, the delivery device 1010 may be advanced toward the aortic valve AV, for example via a retrograde transfemoral route, until the distal sheath 1024 is near or within the native aortic valve AV. As represented by step 5200b, the alignment wires 1090a-1090b may then be advanced to exit the distal sheath 1024, at which point the alignment wires 1090a-1090b may begin to revert to their set shape and curve in a desired direction back upwardly toward the native commissures of the mitral valve MV. In some examples, as represented by optional step 5300b, the position of the alignment wires 1090a-1090b are viewed under imaging, for example under fluoroscopy if the alignment wires 1090a-1090b are radiopaque. Further, the distal sheath 1024 may be actively rotated, as represented by optional step 5400b, to help align and guide the alignment wires 1090a-1090b until they contact the native commissures, as best shown in Fig. 6C. Once the desired contact is confirmed, for example under visualization, the commissures of the prosthetic heart valve will already be aligned with the commissures of the native aortic valve AV. Once the desired positioning and/or centering is achieved, as represented by step 5500b, the prosthetic heart valve may be deployed, for example by retracting the distal sheath 1024 relative to the prosthetic heart valve, either before, after, or simultaneous with retracting the alignment wires 1090a-1090b back into the distal sheath 1024. Also similar to the single alignment wire 1090 embodiment, the force on the two alignment wires 1090a-1090b may help push the distal sheath 1024 toward the center of the native aortic valve AV.

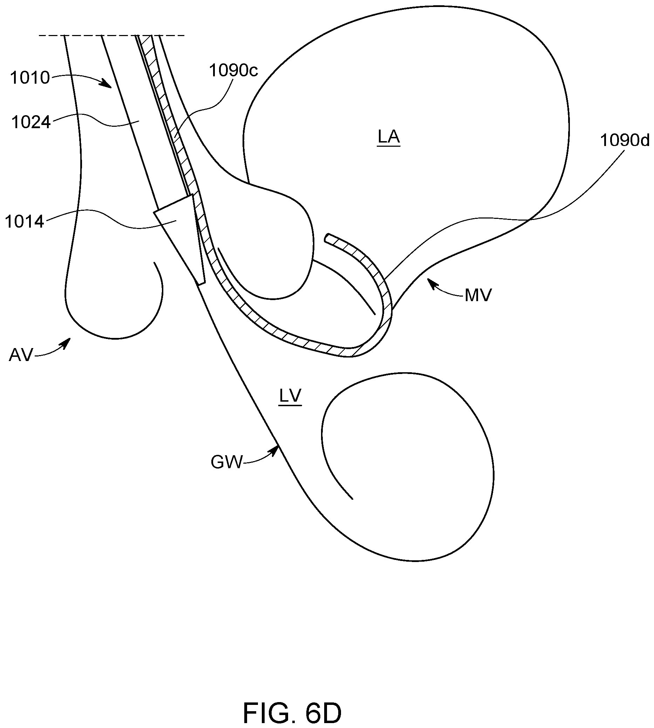

Referring in addition to Fig. 6D, Fig. 6D illustrates an example of a configuration that is structurally similar to the configuration shown in Figs. 6A-6B, with one main difference being that the alignment wire 1090c is shape-set to have a large looped section 1090d that is configured to pass through the mitral valve MV and a distance into the left atrium LA. In some examples, the alignment wire 1090c may be physically similar or identical to alignment wire 1090, with the only exception being the shape to which the alignment wire 1090c is set. In other words, alignment wire 1090 may be shape set so that, as it returns to its set curvature, it tends to contact the outflow surface of the anterior leaflet AL near its base. Alignment wire 1090c, on the other hand, may be shape set with a larger loop so that, as it returns to its set curvature, it passes through the native mitral valve and hooks back toward the atrial septum in one embodiment. In some examples, the alignment wire 1090c may pass through an interior lumen of the delivery device 1010 and, during use, exit through an aperture 1064 in the same fashion as described in connection with Figs. 6A-6B. However, in other examples, the alignment wire 1090c may be received in a separate lumen that rides along the outer diameter of the distal sheath 1024, including a lumen in a separate device that is coupled to the delivery device 1010, for example via a rapid exchange-like lumen. Further, Fig. 6D illustrates the delivery device riding over a guidewire GW. It should be understood that any of the embodiments described herein may include a guidewire GW that extends through a guidewire lumen of the delivery device 1010 to assist with delivery.

One main benefit of having the alignment wire 1090c shape set to a large loop that can be directed by the delivery device 1010 to curl around the mitral valve MV, and particularly the anterior leaflet AL thereof, is that the alignment wire 1090c may provide for a pulling force that brings the distal sheath 1024 toward the center of the native aortic valve AV. While the embodiments described in connection with Figs. 6A-6C noted that the alignment wires may provide a pushing force to push the distal sheath 1024 toward the center of the aortic valve AV, in some examples of delivery device 1010, the tendency is for the distal sheath 1024 to be positioned away from the mitral valve MV toward the right coronary artery RCA. Thus, in some examples, it may be more helpful to have a pulling force that can move the distal sheath 1024 closer to the mitral valve MV, compared to a pushing force that can move the distal sheath 1024 farther away from the mitral valve MV. In addition to this pulling force assisting with centering of the distal sheath, the alignment wire 1090c may be used for commissure alignment in substantially the same way described in connection with Fig. 6B, except that the alignment wire 1090c wraps around the anterior leaflet AL instead of contacting the base of the anterior leaflet AL on its outflow side.

As with the embodiment of Figs. 6A-C, for the example shown in Fig. 6D, the prosthetic heart valve (*e.g.,* prosthetic heart valve 200 of Fig. 2A), may be mounted in or on the delivery device 1010 in a pre-determined rotational orientation (as represented by step 5000c in the flow chart of Fig. 6G) so that, when the alignment wire 1090c loops through the native mitral valve MV and back toward the atrial septum, the commissures of the prosthetic heart valve are in rotational alignment with the commissures of the native aortic valve AV. In step 5100c, after loading the prosthetic heart valve into or onto the delivery device 1010 in the desired orientation, the delivery device 1010 may be advanced toward the aortic valve AV, for example via a retrograde transfemoral route, until the distal sheath 1024 is near or within the native aortic valve AV. As represented by step 5200c, the alignment wires 1090c may then be advanced to exit the distal sheath 1024, at which point the alignment wire may begin to revert to its set shape and curve in a desired direction back upwardly through the native mitral valve and back toward the atrial septum. In some examples, as represented by optional step 5300c, the position of the alignment wire 1090c is viewed under imaging, for example under fluoroscopy if the alignment wire 1090c is radiopaque. Further, the distal sheath 1024 may be actively rotated, as represented by optional step 5400c, to help align and guide the alignment wire 1090c until its passes through the native mitral valve MV and hooks back toward the atrial septum and is in contact with the inflow surface of the anterior leaflet AL, as best shown in Fig. 6D. Once the desired contact is confirmed, for example under visualization, the commissures of the prosthetic heart valve will already be aligned with the commissures of the native aortic valve AV. Once the desired positioning and/or centering is achieved, as represented by step 5500c, the prosthetic heart valve may be deployed, for example by retracting the distal sheath 1024 relative to the prosthetic heart valve, either before, after, or simultaneous with retracting the alignment wire 1090c back into the distal sheath 1024.

Although some of the embodiments described above are generally directed to leveraging positions of the vessel branches relative to the native aortic valve or leveraging the positions of the aortic and/or mitral valve to assist with commissure alignment and/or centering of the delivery device within the native aortic valve, in other embodiments, features on the prosthetic heart valve itself may be used to assist in commissure alignment.

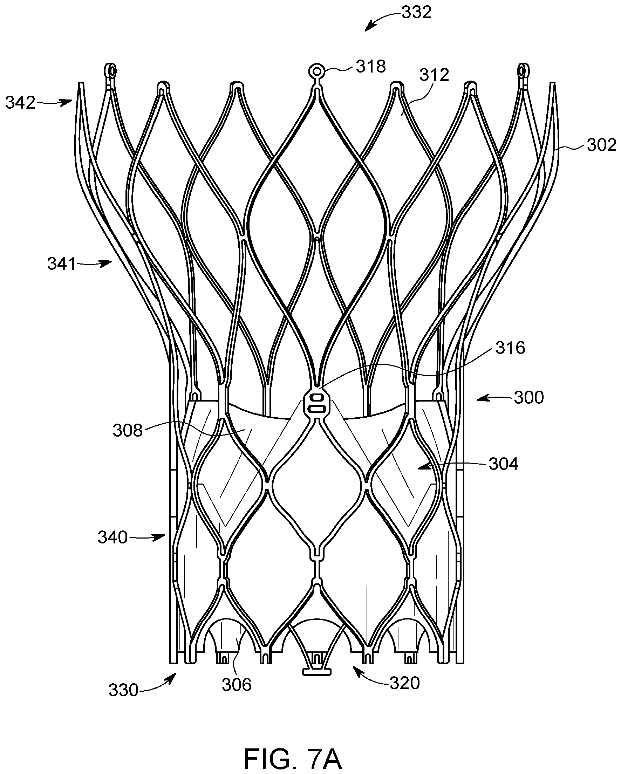

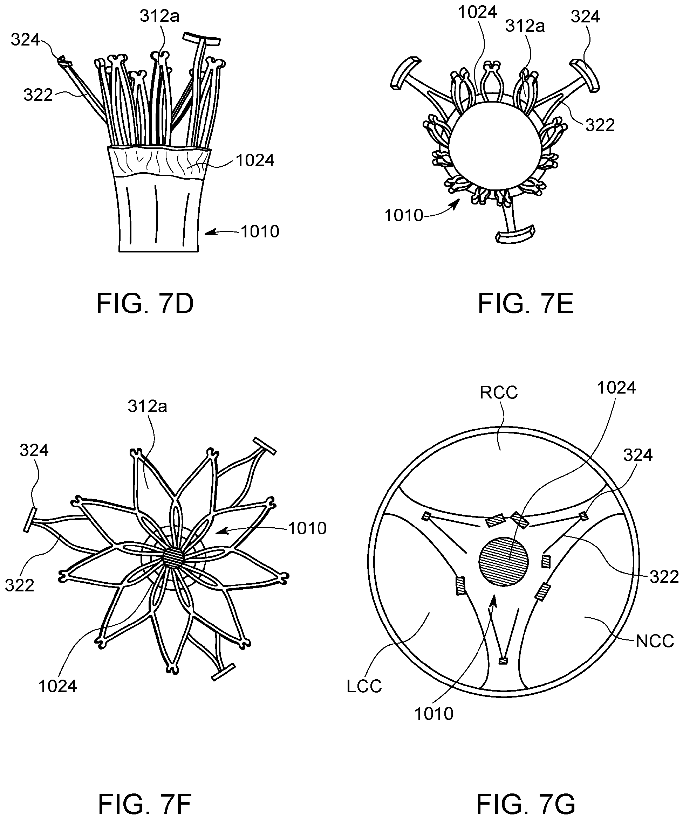

Referring now in addition to Fig. 7A, Fig. 7A illustrates an example of a prosthetic heart valve 300,. Prosthetic heart valve 300 may be similar or identical to prosthetic heart valve 200 described above, with certain exceptions described below. In the illustrated example, the frame 302 may be formed from any biocompatible material, such as metals, metal alloys, synthetic polymers or biopolymers capable of functioning as a frame. Frame 302 may extend from an inflow or annulus end 330 to an outflow or aortic end 332, and may include an annulus section 340 adjacent the inflow end and an aortic section 342 adjacent the outflow end. In some examples, the annulus section 340 has a relatively small cross-section in the expanded condition, while in some examples, the aortic section 342 has a relatively large cross-section in the expanded condition. In one example, annulus section 340 is in the form of a cylinder having a substantially constant diameter along its length. However, in other examples, annulus section 340 may have other shapes when expanded, including frustoconical, bulbous, *etc.* A transition section 341 in some examples tapers outwardly from the annulus section 340 to the aortic section 342. Each of the sections of the frame 302 may include a plurality of cells 312 connected to one another in one or more annular rows around the frame, although other configurations may be suitable in other examples. As shown in the example of Fig. 7A, the annulus section 340 may have two annular rows of complete cells 312 and the aortic section 342 and transition section 341 may each have one or more annular rows of cells 312. The cells 312 in the aortic section 342 may be larger than the cells 312 in the annulus section 340 in some examples. The larger cells in the aortic section 342 may better enable the prosthetic valve 300 to be positioned without the frame structure 302 interfering with blood flow to the coronary arteries. In the illustrated example, cells 312 are generally diamond-shaped when the frame 302 is in the expanded condition, but it should be understood that the cells in other examples may have other shapes (*e.g.,* chevrons, hexagons, *etc*.)*.*

In some examples, frame 302 includes one or more retaining elements 318 at the outflow end 332 thereof, the retaining elements being sized and shaped to cooperate with complementary retaining structures provided on the deployment device. When included, the engagement of retaining elements 318 with the complementary retaining structures on the deployment device may help maintain prosthetic heart valve 300 in an assembled relationship with the deployment device, may minimize longitudinal (*e.g.* axial) movement of the prosthetic heart valve relative to the deployment device during unsheathing or re-sheathing procedures, and/or may help prevent rotation of the prosthetic heart valve relative to the deployment device as the deployment device is advanced to the target location and during deployment.

The example of prosthetic heart valve 300 illustrated in Fig. 7A may include a valve assembly 304. The valve assembly 304 may include a skirt or cuff 306 and a plurality of prosthetic leaflets 308 which collectively function as a one-way valve. The valve assembly 204 described in connection with prosthetic heart valve 200 above, including alternate options described therewith, may apply to the valve assembly 304 of prosthetic heart valve 300. The commissures between adjacent prosthetic leaflets may be connected to corresponding commissure features 316 on frame 302. The example of prosthetic heart valve 300 shown in Fig. 7A is configured for use with three prosthetic leaflets 308, as well as three commissure features 316. As can be seen in Fig. 7A, in this example, the commissure features 316 may lie at the intersection of four cells 312, two of the cells being adjacent one another in the same annular row, and the other two cells being in different annular rows and lying in end-to-end relationship. However, in other examples, commissure features 316 may be provided at different locations or in different configurations, or even omitted entirely. In some examples, commissure features 316 are positioned entirely within annulus section 340 or at the juncture of annulus section 340 and transition section 341. In the illustrated example, commissure features 316 include one or more eyelets which facilitate the suturing of the leaflet commissure to the frame 302. However, it should be appreciated that the prosthetic heart valve may have more or fewer prosthetic leaflets and commissure features. For example, an alternative prosthetic heart valve (*e.g.,* a prosthetic mitral valve) may include two prosthetic leaflets with two commissures.

In the illustrated example, prosthetic heart valve 300 may be a self-expanding prosthetic heart valve, which may include a frame 302 formed of a shape-memory alloy such as a nickel-titanium alloy (including nitinol). The alignment features described below in connection with frame 302 may be best suited for use in a self-expanding prosthetic heart valve, but it should be understood that these alignment features may be useful in other types of prosthetic heart valves, including mechanically-expandable valve and balloon-expandable valves.

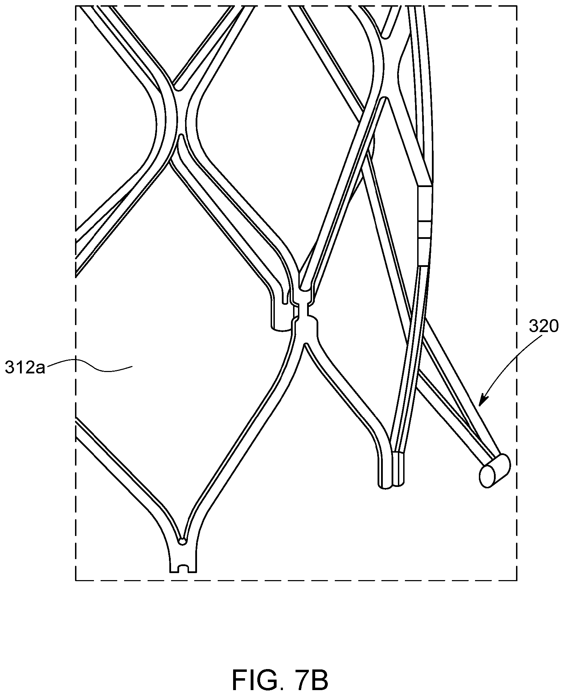

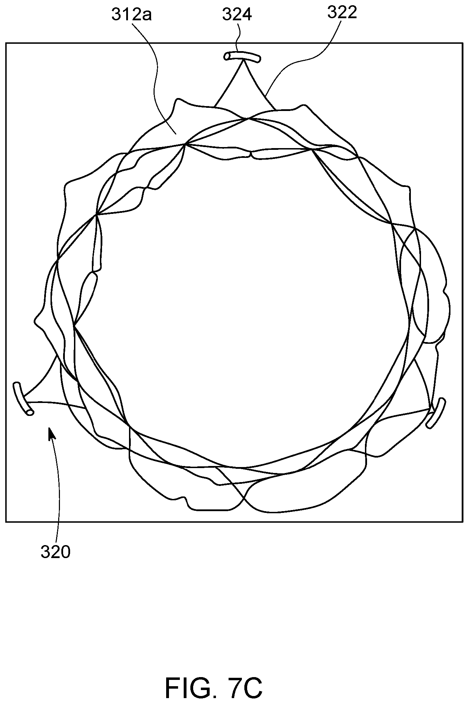

Referring now in addition to Figs. 7B-7C, Fig. 7B is an enlarged view of the inflow end 330 of frame 302, and Fig. 7C is an end view of the inflow end 330 of frame 302. The valve assembly 304 is omitted from the views of Figs. 7B-7C for ease of illustration. Referring to Figs. 7A-7C, the illustrated example of frame 302 includes an inflow-most row of complete cells 312a. The frame 302 may additionally include commissure alignment features 320. In the illustrated example, the commissure alignment features 320 are formed integrally with the frame, although in other examples, the commissure alignment features 320 may be formed separately and then coupled to the frame 302. Preferably, although not necessarily, the frame 302 includes a total number of commissure alignment features 320 that is equal to the number of prosthetic leaflets in the prosthetic heart valve 300 and/or equal to the number of the commissure attachment features 316 on frame 302. In this particular example, each commissure alignment features 320 includes two struts 322 that generally form a half-diamond. For example, as best shown in Figs. 7A and 7C, each strut 322 may have a first end coupled to a strut of an inflow-most cell 312a, for example the two struts 322 may be coupled to midpoints of struts of circumferentially adjacent inflow-most cells 312a. For each commissure alignment features 320 of this example, the two struts 322 may have second ends that join together at an apex. In the illustrated example, a horizontal or circumferential strut 324 is positioned at the apex to form a general "T"-shape end.

In the illustrated example, each of the three commissure alignment features 320 are preferably positioned at substantially equal intervals (*e.g.* at 120 degree intervals +/- 5 degrees) around the circumference of the inflow end 330 of the frame 302. In some examples, each commissure alignment feature 320 is positioned in longitudinal alignment (*e.g.* +/- 5 degrees) with a corresponding one of the commissure attachment features 316, and therefore in longitudinal alignment with a corresponding commissure of the prosthetic valve mounted within the frame 302. However, in other embodiments, the commissure alignment features 320 may be positioned in longitudinal alignment with portions of the frame 302 between circumferentially adjacent pairs of commissure attachment features 316.

As best shown in Figs. 7A-7C, while the annulus section 340 of the frame 302 may be shape set to have a generally cylindrical (or conical or other suitable shape) when in the expanded condition in the absence of applied forces, the commissure alignment features 320 are preferably shape-set so that they extend radially outwardly from the annulus section 340 (and particularly the inflow end 330 of the frame 302) in the absence of applied forces. For example, as best shown in Figs. 7A-7C, the struts 322 of each commissure alignment feature 320 may be shape set so that the struts 322 extend outwardly between their connection to cells 312a in a direction toward the horizontal strut 324. Although the commissure alignment features 320 may be biased radially outwardly from the inflow end 330 of the frame 302 in the absence of applied forces, relatively small inward forces, for example resulting from contact with tissue during self-expansion, may force the commissure alignment features 320 to press radially inwardly so that they will be in general alignment with the profile of the remainder of annulus section 340 following implantation. In other words, the commissure alignment features 320 may be highly compliant in various examples of the disclosure.

Referring now in addition to Figs. 7D-7E, Figs. 7D-7E are side and end views, respectively, of frame 302 (with the valve assembly 304 omitted for easy of illustration) in the initial stages of self-expansion as the inflow end 330 of the frame 302 exits the distal sheath 1024 of a delivery device. It should be understood that the delivery device 1010 shown in Figs. 7D-7G may be the same or similar to the delivery device 1010 shown in Fig. 2A. However, it should also be understood that the delivery device 1010 shown in Figs. 7D-7G does not need to include all features of the delivery device 1010 shown and described in connection with Fig. 2A, and similarly the delivery device 1010 shown in Figs. 7D-7G may include features in addition to those shown and described in connection with Fig. 2A. As can be seen in Figs. 7D-7E, as the constraint of the distal sheath 1024 is removed from the inflow end 330 of the frame 302, the inflow end of the frame 302 begins to self-expand. However, in some examples, the commissure alignment features 320 (particularly the horizontal struts 324 thereof), extend farther radially outwardly than the inflow-most cells 312a during this early stage of deployment. Preferably, some or all of the components of the commissure alignment features 320, for example at least the horizontal struts 324, are radiopaque so that they are readily identifiable and distinguishable from the remainder of the frame 302 during deployment. In some examples, the deployment may be visualized (e.g. under fluoroscopy) during deployment to identify the location of the three commissure alignment members 320. If the three commissure alignment members 320 are in longitudinal alignment with the commissure attachment features 316, the prosthetic heart valve 300 may be actively or passively rotated until the commissure alignment members 320 are rotationally aligned with the commissures of the native aortic valve so achieve commissure alignment between the prosthetic heart valve 300 and the native aortic valve AV. If the three commissure alignment members 320 have a different, known rotational offset to the commissure attachment features 316, the known offset may be used to achieve alignment between the commissures of the prosthetic heart valve 300 and those of the native aortic valve AV. Once the desired rotational alignment is achieved, deployment of the prosthetic heart valve 300 may be completed, according to various examples of the disclosure.

In some examples, the commissure alignment features 320 may be used only as visual indicators to assist with achieving commissure alignment. However, in some examples, the commissure alignment features 320 may physically assist with achieving commissure alignment. In examples in which the commissure alignment features 320 are longitudinally aligned with the prosthetic commissures, the commissure alignment features 320 may physically nest within the native commissures to help achieve commissure alignment. For example, Fig. 7F illustrates the frame 302 in a further state of deployment compared to Figs. 7D-7E, with the commissure alignment features 320 extending farther radially outwardly compared to Figs. 7D-7E. Fig. 7G illustrates a highly schematic representation of the deployment stage shown in Fig. 7F with the frame 320 within the native aortic valve AV. In some examples, active rotation may be utilized to rotate the frame 302 until the commissure alignment features 320 (*e.g.* horizontal struts 324 and/or struts 322) are physically nested in the native commissures between pairs of adjacent leaflets of the native aortic valve AV. In other examples, passive rotation may be utilized so that, as the leaflets of the native aortic valve AV close over the commissure alignment features 320, the force from the coapting leaflets may force the commissure alignment features 320 to rotate to nest within the native commissures, thus achieving commissure alignment. This passive alignment may be generally similar to the passive alignment described above in connection with Figs. 5A-5B, except that the leaflets are acting directly on the prosthetic heart valve 300 instead of directly acting on the delivery device 1010. In this example, once the commissure alignment features 320 are confirmed to be nested within the native commissures, the prosthetic heart valve 300 may be fully deployed from the distal sheath 1024 with the commissures of the prosthetic heart valve 300 being in rotational alignment with the commissures of the native aortic valve AV.

It should be understood that the commissure alignment features 320 shown in connection with prosthetic heart valve 300 are merely one example of a suitable shape and/or geometry. Various other shapes, including those the omit the horizontal strut 324, shapes that or alternatives to the horizontal strut 324 and/or two struts 322, *etc.* may be suitable for achieving similar results. Further, as noted above, in some examples the commissure alignments features 320 may be compliant enough so that, after contact with tissue (*e.g.* the native commissures) upon complete or mostly complete expansion, the commissure alignment features 320 take the general shape of the tissue which they contact. This may be useful, for example, to prevent the commissure alignment features 320 from causing trauma to the native tissue. In some examples, the commissure alignment features 320 may serve purposes in addition to, or alternative to, commissure alignment. For example, the commissure alignment features 320 may assist with depth control. In this context, "depth" refers to the positioning of the prosthetic heart valve in the direction of blood flow (*e.g.* farther into the left ventricle or farther into the aorta). By positioning the commissure alignment features 320 at the desired location with respect to the native commissures, the depth of the prosthetic heart valve 300 within the native aortic valve will also be known. Thus, commissure alignment features 320 may provide a depth control functionality that may be separate from the commissure alignment functionality.

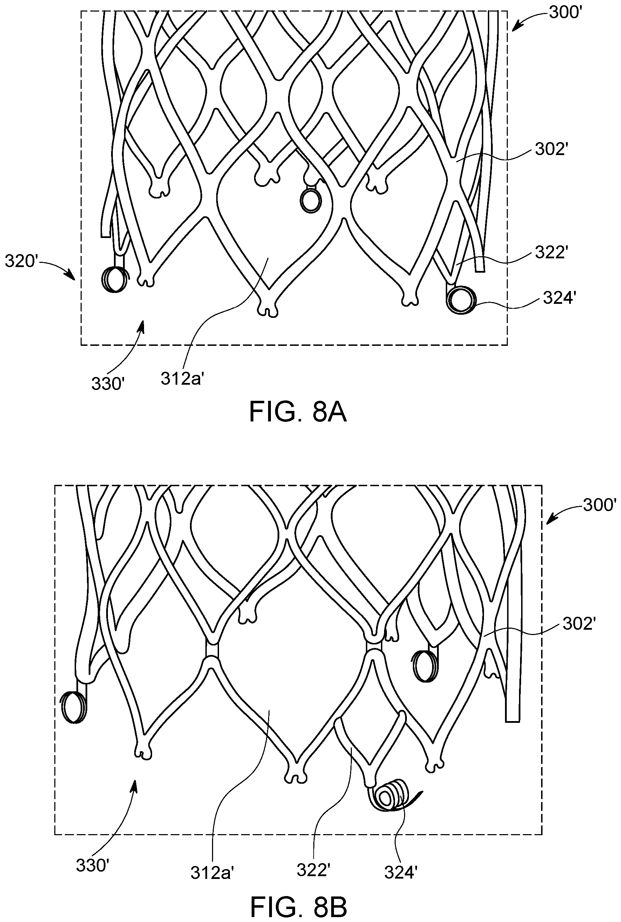

Referring now in addition to Figs. 8A-8B, Figs. 8A-8B illustrate the inflow end 330' of a frame 302' of a prosthetic heart valve 300' that may, in one example, be identical to frame 302 of prosthetic heart valve 300 except for the configuration of commissure alignment features 320'. Prosthetic heart valve 300' is shown in Figs. 8A-8B without a valve assembly for east of illustration, but it should be understood that the prosthetic heart valve 300' may include a valve assembly similar or identical to valve assembly 204 or 304. Commissure alignment features 320' may, in one example, be similar or identical to commissure alignment features 320 in structure and function with the exception of the apex geometry. For example, commissure alignment feature 320' may include two struts 322' that each have a first end coupled to a midpoint of a strut of respective adjacent inflow-most cells 312a'. The two struts 322' may include second ends that form an apex, but instead of a horizontal strut 324 being positioned at the apex, a coil 324' may be formed at the apex. In one example, the coil 324' may be a single wire that extends from the apex, and which is coiled into one or more loops. In some examples, in the absence of applied force to the frame, the coil 324' may be positioned radially outward of the inflow-most cells 312a'. In some examples, in the absence of applied force to the frame, the coil 324' may loop around a coil axis that extends in a direction transverse to the central longitudinal axis of the frame.

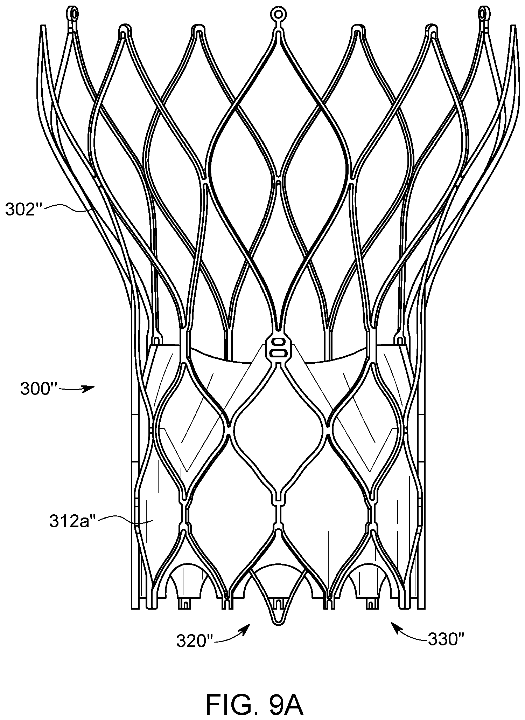

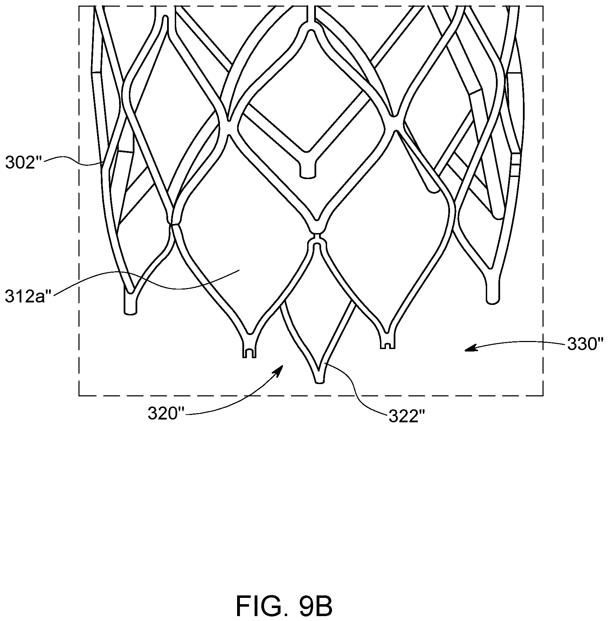

Referring now in addition to Figs. 9A-9B, Fig. 9A shows a prosthetic heart valve 300" including frame 302". In one example, prosthetic heart valve 300" (and frame 302") may be identical to prosthetic heart valve 300 (and frame 302) except for the configuration of commissure alignment features 320". Fig. 9B shows an enlarged view of the inflow end 330" of frame 302", with other components of the prosthetic heart valve 300" (such as the valve assembly) omitted for ease of illustration. Commissure alignment features 320" may, in one example, be similar or identical to commissure alignment features 320 in structure and function with the exception of the apex geometry. For example, commissure alignment feature 320" may include two struts 322" that each have a first end coupled to a midpoint of a strut of respective adjacent inflow-most cells 312a". The two struts 322' may include second ends that form an apex, but the horizontal strut 324 is omitted.

Other than the different geometries of commissure alignment features 320' and 320", the structure and function of the commissure alignment features may, in one example, be identical in configuration and function described in connection with commissure alignment features 320, including the various alternatives described in connection with commissure alignment features 320.

Although various individual features for commissure alignment are described herein, it should be understood that some of the features may be combinable into a single system. For example, the commissure alignment features on the prosthetic heart valve (*e.g.* as shown in Figs. 7A-9A) may be combined with delivery device features for commissure alignment (*e.g.* as shown in Figs. 3A-6D). In some examples, multiple commissure alignment features disclosed in connection with the delivery system may be used with each other, for example alignment wires to cannulate one or more aortic head vessels may be combined with alignment wires to cannulate one or more coronary arteries and/or with alignment wires that interact with the mitral valve and/or with alignment wires that interact with the aortic valve leaflets (or commissures).

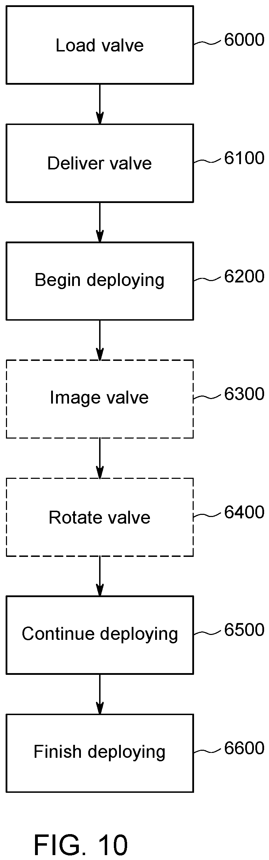

Fig. 10 illustrates a flow chart showing an example of a method of use relating to the commissure alignment features of Figs. 7A-9B. For example, in a first step 6000, the prosthetic heart valve (*e.g.* prosthetic heart valve 300, 300' or 300") may be loaded into a delivery device (*e.g.* delivery device 1010) in a collapsed condition, for example with retainers (*e.g.* retainers 318) received within complementary retainers (*e.g.* retainers 1025) of the delivery device. The prosthetic heart valve may be advanced into or adjacent a patient's native heart valve (*e.g.* aortic valve, mitral valve, pulmonary valve, or tricuspid valve) in step 6100, for example via a retrograde transfemoral approach (which may be particularly suited for aortic valve treatment). The user may initiate deployment of the inflow end (e*.g.* inflow end 330, 330', or 330") of the prosthetic heart valve in step 6200, for example by retracting an overlying sheath (*e.g.* distal sheath 1024) and allowing self-expansion of the prosthetic heart valve to begin. In optional step 6300, the prosthetic heart valve may be imaged, for example using fluoroscopy, to determine the orientation of the commissure alignment features (*e.g.* commissure alignment features 320, 320', or 320"). In optional step 6400, the prosthetic heart valve may be rotated, for example by rotating the delivery device or a component thereof, to align the commissure alignment features with the commissures of the native heart valve. It should be understood that steps 6200 and 6300, if one or both are performed, may be performed multiple times and in different order than shown in the flow chart of Fig. 10. In step 6500, deployment may continue until the commissure alignment features contact corresponding ones of the native commissures, which may help confirm one or both of rotational alignment and desired depth of the prosthetic heart valve. In step 6600, deployment may be completed and the prosthetic heart valve may be fully deployed into the native heart valve.

Although the invention herein has been described with reference to particular embodiments, it is to be understood that these embodiments are merely illustrative of the principles and applications of the present invention. It is therefore to be understood that numerous modifications may be made to the illustrative embodiments and that other arrangements may be devised without departing from the scope of the present invention as defined by the appended claims. It will be appreciated that the various dependent claims and the features set forth therein can be combined in different ways than presented in the initial claims. It should also be appreciated that the features described in connection with individual embodiments may be shared with others of the described embodiments.

## Claims

1. A prosthetic heart valve system comprising:
a delivery device (1010) including a catheter, the catheter including an aperture (1064) in a sidewall thereof;
a collapsible and expandable prosthetic heart valve (200) including a frame (202) and a plurality of prosthetic leaflets (208) mounted within the frame, the prosthetic heart valve configured to be mounted on or within the catheter in a collapsed condition for transcatheter delivery to a patient; and
an alignment wire (1060) configured to be positioned within the catheter during transcatheter deliver to the patient, the alignment wire being formed of a shape memory material and being shape-set so that, while the alignment wire is within the catheter it is substantially straight and after being advanced through the aperture, a distal portion of the alignment wire extending beyond the aperture is biased at an angle of about 90 degrees relative to an intermediate portion of the alignment wire remaining within the catheter.

2. The prosthetic heart valve system of claim 1, wherein the delivery device (1010) includes a handle (1020) at a proximal end thereof, and an actuator operatively coupled to the handle, wherein actuation of the actuator rotates the catheter so that, when the prosthetic heart valve (200) is mounted on or within the catheter, rotation of the catheter causes rotation of the prosthetic heart valve.

3. The prosthetic heart valve system of claim 1, wherein the alignment wire (1060) is formed of nitinol.

4. The prosthetic heart valve system of claim 1, wherein the alignment wire (1060) is inflatable.

5. The prosthetic heart valve system of claim 1, wherein the alignment wire (1060) is ribbon-shaped with a substantially rectangular cross-section.

6. The prosthetic heart valve system of claim 1, wherein the prosthetic heart valve (200) is a self-expanding, and the catheter includes an outer shaft (1022) and a distal sheath (!024) positioned distal to the outer shaft, the distal sheath having a diameter that is larger than a diameter of the outer shaft, the prosthetic heart valve configured to be mounted within the distal sheath of the catheter during transcatheter delivery to the patient.

7. The prosthetic heart valve system of claim 6, wherein the aperture (1064) is positioned on the distal sheath (1024) so that when a portion of the distal sheath is positioned within a native aortic valve annulus of the patient, the aperture is substantially axially aligned with an ostium of a left coronary artery (LCA) or an ostium of a right coronary artery (RCA).

8. The prosthetic heart valve system of claim 6, wherein the aperture (1064) is positioned on the outer shaft (1022) so that when a portion of the distal sheath (1024) is positioned within a native aortic valve annulus of the patient, the aperture is substantially axially aligned with an ostium of a brachiocephalic artery (BA), an ostium of a left common carotid artery (LCCA), or an ostium of a left subclavian artery.

9. The prosthetic heart valve system of claim 1, wherein the sidewall of the catheter includes a lumen (1062) extending axially therethrough, the alignment wire (1060) being configured to be received through the lumen.

10. The prosthetic heart valve system of claim 9, wherein the lumen (1062) terminates in the aperture (1064).
